(19) Europäisches Patentamt
European Patent Office
Office européen des brevets

(11) Publication number: **0 195 817 B1**

(12) # EUROPEAN PATENT SPECIFICATION

(45) Date of publication of patent specification: **18.10.89**

(51) Int. Cl.[4]: **C 07 K 5/06,** A 61 K 37/02

(21) Application number: **85905015.5**

(22) Date of filing: **19.09.85**

(86) International application number: **PCT/US85/01778**

(87) International publication number: **WO 86/01803 27.03.86 Gazette 86/07**

(54) **CARBOXYALKYLDIPEPTIDES, THEIR PREPARATION AND PHARMACEUTICAL COMPOSITIONS CONTAINING THEM.**

(30) Priority: **24.09.84 US 653186**

(43) Date of publication of application: **01.10.86 Bulletin 86/40**

(45) Publication of the grant of the patent: **18.10.89 Bulletin 89/42**

(84) Designated Contracting States: **AT BE CH DE FR GB IT LI LU NL SE**

(56) References cited:
**EP-A-0 088 350**
**US-A-4 468 396**

**Chemical Abstracts, Vol. 97, no. 13, 1982, Columbus, Ohio, (US). A.M. El-Naggar et al.:"Synthesis of some biologically active visnagin-9-sulfonyl amino acid and dipeptide derivatives", see page 651, abstract no. 110378q**

(73) Proprietor: **SCHERING CORPORATION**
**2000 Galloping Hill Road**
**Kenilworth New Jersey 07033 (US)**

(72) Inventor: **ANDREWS, David, Roy**
**22 Ridge Avenue**
**Bloomfield, NJ 07003 (US)**
Inventor: **GAETA, Federico, Carlos, Arejola**
**5 Shawnee Avenue**
**Rockaway Township, NJ 07866 (US)**

(74) Representative: **von Kreisler, Alek, Dipl.-Chem. et al**
**Deichmannhaus am Hauptbahnhof**
**D-5000 Köln 1 (DE)**

**EP 0 195 817 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel carboxyalkyldipeptides, to their preparation and to pharmaceutical compositions containing them. More particularly, this invention relates to carboxyalkyldipeptides in which the dipeptide moiety is joined through a sulfonamido group to a diuretic moiety. The compounds of the present invention exhibit angiotensin converting enzyme inhibitory activity and/or diuretic activity. Compounds of this invention are indicated as being useful in the treatment of hypertension, congestive heart failure and glaucoma.

EP—A—0 088 350 relates to carboxyalkyl-dipeptides having partly similar substituents. The compounds of said document are also useful as antihypertensive agents, in the treatment of congestive heart failure and glaucoma.

In accordance with one of its aspects, the present invention provides novel compounds of the general formula I

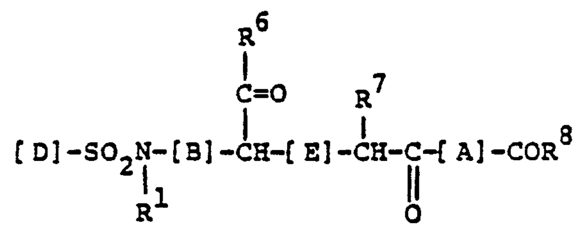

I

and the pharmaceutically acceptable salts thereof, wherein:

A is

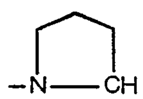 , 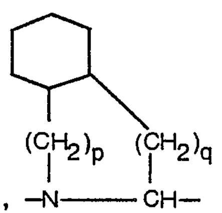 , 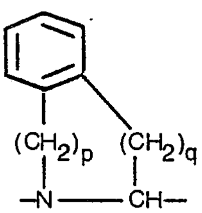 , 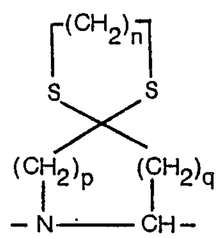 or 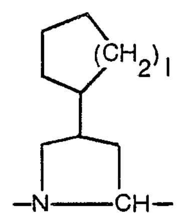

IIa IIb IIc IId IIe

l is 1 or 2;

n is 0 or 1;

p and q are 0, 1 or 2, provided that in structures IIb and IIc the sum of p and q is 1 or 2, and that in formula IId, p is not 0;

B is —[J]—[L]—[M]—;

D is

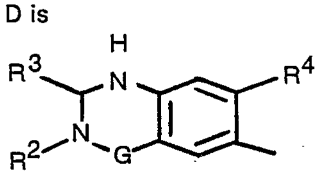

III

or

E is —NH—, —O—, —S—, or $-CH_2-$;

G is $-\overset{\overset{O}{\|}}{C}-$ or $-SO_2-$;

J is $-(CH_2)_s-$ or $-((:CH_2)_t-W)-$;

L is a chemical bond, cis- or trans $C_2-C_6$-alkenylene, $C_2-C_6$-alkynylene, —Z-aryl-, -aryl-Z—, —Z-cycloalkyl-, -cycloalkyl-Z—, a 5- or 6-membered heterocyclic radical comprising 3 to 5 carbon atoms and 1 or 2 heteroatoms selected from N, O and S, or a $R^5$-substituted heterocyclic radical, having one or more heteroatoms selected from N, O or S, wherein aryl is

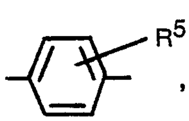 , 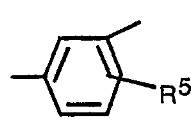 or 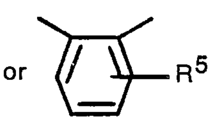

and cycloalkyl is

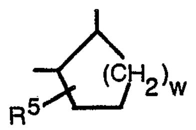 , 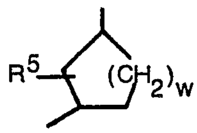 or 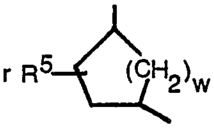

wherein

w is 1, 2 or 3;

M is $(CH_2)_u$— or —$((CH_2)_t$-X—$(CH_2)_v)$—;

W is $—\overset{O}{\overset{\|}{C}}NH—$ or $—NH\overset{O}{\overset{\|}{C}}—$;

X and Z are independently a chemical bond, —$NR^9$—, —O—, —S—, $—\overset{O}{\overset{\|}{C}}NH—$ or $—NH\overset{O}{\overset{\|}{C}}—$;

s, u and v are independently 0—5;

t is 1—5;

$R^1$, $R^2$ and $R^9$ are independently hydrogen, $C_1$—$C_6$-alkyl or $C_1$—$C_6$-acyl;

$R^3$ is hydrogen, $C_1$—$C_6$-alkyl, halo-$C_1$—$C_6$-alkyl (in particular halo-, and di-halo-$C_1$—$C_6$-alkyl) phenyl-$C_1$—$C_6$-alkyl, (cycloalyl)-$C_1$—$C_6$-alkyl, aminomethyl, $C_1$—$C_6$-alkylaminomethyl, phenyl-$C_1$—$C_6$-alkylaminomethyl, (cycloalkyl)-$C_1$—$C_6$-alkylaminomethyl, $C_1$—$C_6$-alkylthiomethyl, halo-$C_1$—$C_6$-alkylthiomethyl, 2-, 3- or 4-pyridyl-$C_1$—$C_6$-alkyl, 2-, 4- or 5-thiazolyl-$C_1$—$C_6$-alkyl, 2-, 4- or 5-1*H*-imidazolyl-$C_1$—$C_6$-alkyl, 1-imidazolyl-$C_1$—$C_6$-alkyl, 1-morpholino-$C_1$—$C_6$-alkyl or hydroxyphenyl-$C_1$—$C_6$-alkyl;

$R^4$ is chlorine or $CF_3$;

$R^5$ is hydrogen, halogen, $C_1$—$C_6$-alkyl, $C_1$—$C_6$-acyl, $C_1$—$C_6$-alkoxy, halo-$C_1$—$C_6$-alkyl or phenyl-$C_1$—$C_6$-alkyl;

$R^7$ is hydrogen, $C_1$—$C_6$-alkyl or amino-$C_1$—$C_6$-alkyl;

$R^6$ and $R^8$ are independently hydroxy, alkoxy having from 1 to 8 carbon atoms, benzyloxy, allyloxy, $R^{10}$-$Q_r$—$(CH_2)_m$—O—, wherein Q is oxygen or sulfur, r is 0 or 1 and m is 2 to 4,

$$—O\overset{R^{11}}{\overset{|}{C}}H—OCO\text{-alkyl}$$

wherein the alkyl has from 3 to 8 carbon atoms,

$$—O\overset{R^{11}}{\overset{|}{C}}HCO\text{-phenyl}$$

wherein the phenyl may be substituted with group T defined below, 1-glyceryl,

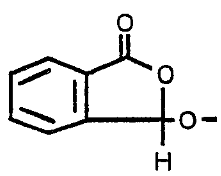 or 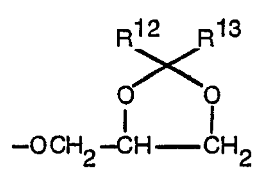

$R^{10}$ is phenyl, substituted phenyl wherein the substituents are chosen from group T, 1-naphthyl or 2-naphthyl;

T is halogen, hydroxy, trifluoromethyl, $C_1$—$C_6$-alkoxy, $C_1$—$C_6$-alkyl, 2-furanyl, 3-furanyl, 2-thienyl, 3-thienyl, phenyl and substituted phenyl wherein the substituents are chosen from halogen, hydroxy, trifluoromethyl, $C_1$—$C_6$-alkoxy or $C_1$—$C_6$-alkyl;

$R^{11}$ is hydrogen or alkyl having from 1 to 8 carbon atoms;

$R^{12}$ is hydrogen, $C_1$—$C_6$-alkyl, unsubstituted or substituted phenyl and substituted or unsubstituted phenyl $C_1$—$C_6$-alkyl wherein phenyl may be substituted by group T;

$R^{13}$ is hydrogen or $C_1$—$C_6$-alkyl;

provided that if L is alkenylene or alkynylene, J is —$(CH_2)_s$— wherein s is 1—5; provided that if L is —Z-aryl- or —Z-cycloalkyl-, J is —$(CH_2)_s$— wherein s is 2—5; provided that if L is alkenylene, alkynylene -aryl-Z— or -cycloalkyl-Z—, M is —$(CH_2)_u$- wherein u is 1—5; provided that if s and u are each zero, L is aryl or cycloalkyl (i.e. Z is a bond); and provided that if s and v are each zero, L is aryl or cycloalkyl (i.e. Z is a chemical bond);

with the further provision that when D is of formula IIIb and $R^1$ is hydrogen, B is not $-(CH_2)_4-$; and that when D is of formula IIIb and $R^1$ is hydrogen or $C_1-C_6$-alkyl, B is not $-(CH_2)_s$-aryl-$(CH_2)_t$-X$-(CH_2)_v-$ wherein s is 0 or 1, t is 1, v is 0 or 2 and X is a chemical bond, —O—, or —S—.

When A is formula IIb or IIc, the preferred sum of p and q is 1; when A is of formula IId, preferred values for each of p and q are 1 and n being zero. Preferably, A is IIa or IIb where p is o and q is 1.

D is preferably of the formula IIIa; $R^4$ being preferably chlorine and G being preferably $-SO_2-$. $R^2$ is preferably hydrogen, and $R^3$ preferably phenylethyl, (cyclopentyl)methyl, chloromethyl, dichloromethyl, 2-pyridinylethyl, butyl, pentyl or trifluoroethylthiomethyl.

For B, preferred values are of the formula

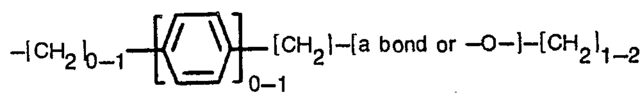

i.e., where J is $-(CH_2)_s-$ and s is 0—1, L is a chemical bond or —Z-aryl- wherein Z is a chemical bond, and M is $-((CH_2)_t-X-(CH_2)_v)$, wherein t is 1, v is 1—2, and X is a chemical bond or —O—.

For compounds wherein L is a heterocyclic radical as defined above thiazolyl rings joined to the rest of molecule at the 2,4 and 2,5 positions are preferred.

A preferred value for E is —NH—.

Preferred values of $R^7$ are hydrogen, methyl and aminobutyl and for $R^6$ and $R^8$ preferred values are hydroxy, ethoxy, methoxy, phenoxyethoxy, 1-glyceryl, pivaloyloxyalkyloxy, and

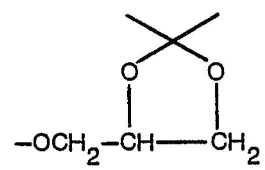

A preferred group of compounds are those represented by the general formula

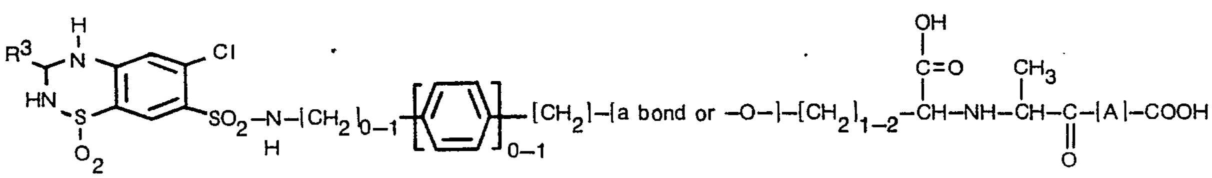

wherein $R^3$ is phenylethyl, chloromethyl, dichloromethyl, butyl, pentyl, (cyclopentyl)methyl, trifluoroethylthiomethyl or 2-pyridinylethyl.

Specifically preferred compounds of the general formula I include:

1-[[2-(S)-[[1(S)-carboxy-5-[[6-chloro-3-chloromethyl-3,4-dihydro-2*H*-1,2,4-benzothiadiazin-7-yl]sulfonylamino]pentyl]amino]-1-oxopropyl]-[2S-(2α, 3aα, 7aα)]-octahydro-1*H*-indole-2-carboxylic acid, *S*,*S*-dioxide,

1-[2-(S)-[[1-(S)-carboethoxy-2-[4-[[[6-chloro-3,4-dihydro-3-(2-phenylethyl)-2*H*-1,2,4-benzothiadiazin-7-yl]sulfonyl-amino]methyl]phenylmethoxy]ethyl]amino]-1-oxopropyl]-[2S-[2α, 3aα, 7aα)]-octahydro-1*H*-indole-2-carboxylic acid, *S*,*S*-dioxide,

1-[2-(S)-[[1-(S)-carboxy-2-[4-[[[6-chloro-3,4-dihydro-3-(2-phenylethyl)-2*H*-1,2,4-benzothiadiazin-7-yl]sulfonylamino]-methyl]phenylmethoxy]ethyl]amino]-1-oxopropyl]-[2S-2α, 3aα, 7aα)]-octahydro-1*H*-indole-2-carboxylic acid, *S*,*S*-dioxide,

N-[2-(*S*)-[[3-[4-[[[6-chloro-3,4-dihydro-3-(2-phenylethyl)-2*H*-1,2,4-benzothiadiazin-7-yl]sulfonylamino]-methyl]phenyl]-1-(*S*)-(ethoxycarbonyl)butyl]amino]-1-oxopropyl]-(*S*)-proline-S,S-dioxide, and

1-[*N*-[3-[4-[[[6-chloro-3,4-dihydro-3-[2-pyridylethyl)-2*H*-1,2,4-benzothiadiazin-7-yl)-sulfonyl)aminomethyl]phenyl]-1(*S*)-(ethoxycarbonyl)-propyl)-(*S*)-alanyl)-cis,synoctahydroindole-2(*S*)-carboxylic acid S,S-dioxide.

As used herein, "$C_1-C_6$-alkyl" means straight or branched chain hydrocarbon radicals of from 1 to 6 carbons, e.g. methyl, ethyl, propyl, isopropyl, butyl, t-butyl, pentyl and hexyl. Similarly, "$C_1-C_6$-alkoxy" means straight or branched alkoxy radicals having from 1 to 6 carbon atoms, e.g. methoxy, ethoxy, propoxy, butoxy, isobutoxy, pentoxy and hexyloxy. "Halogen" means fluorine, chlorine and bromine. "$C_1-C_6$-acyl" means organic radicals obtained by removing the hydroxyl group from the corresponding carboxylic acid of from 1 to 6 carbons, e.g. formyl, acetyl, propionyl and butyryl. "$C_2-C_6$-alkene" means unsaturated hydrocarbon radicals of from 2 to 6 carbon atoms having one double bond, e.g. vinylene propenyl, butenyl, pentenyl and hexenyl, wherein the double bond may be present anywhere in the chain, e.g. 1- or 2-propenyl, 1-, 2- or 3-butenyl. Similarly "$C_2-C_6$-alkyne" means a hydrocarbon radical of from 2 to 6 carbon atoms having one triple bond, e.g. ethynylene, propynyl, butynyl, pentynyl and hexynyl, wherein the triple bond may be present anywhere in the chain, e.g. 1- or 2-propynyl, 1-, 2- or 3-butynyl.

Examples of heterocyclic radicals are:

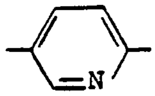 , 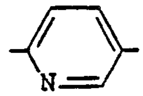 , 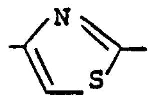 , 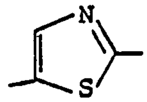 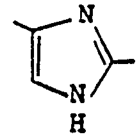 ,

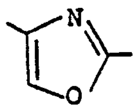 , 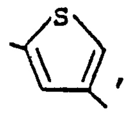 , 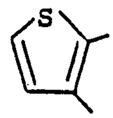 , 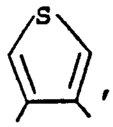 , 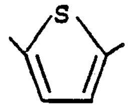

Compounds of the instant invention include various stereoisomers. Preferred stereoisomers are those in which the absolute configuration at carbon atoms adjacent to both a nitrogen and a carbonyl group corresponds most closely to the absolute configuration of L-amino acids.

The compounds of this invention form acid addition salts with various inorganic and organic acids and cationic salts with bases. Cationic salts include ammonium salts, alkali metal salts, e.g. sodium and potassium salts, and alkaline earth metal salts, e.g. calcium and magnesium salts. Salts with organic bases may be prepared, e.g., N-methylglucamine, lysine and arginine. Typically acid additional salts may be formed with such organic and inorganic acids, as HCl, HBr, $H_2SO_4$, $H_3PO_4$, methanesulfonic acid, toluene-sulfonic acid, maleic acid, fumaric acid and camphorsulfonic acid. The non-toxic pharmaceutically acceptable salts are preferred, although other salts are also useful, e.g., in isolating or purifying the products. Acid addition salts (e.g. HCl and maleate) are preferred, especially the hydrochloride.

The salts may be formed by conventional means, as by reacting the free acid or base forms of the product with one or more equivalents of the appropriate base or acid in a solvent or medium in which the salt is insoluble, or in a solvent such as water which is then removed *in vacuo*, or by exchanging the cations of an existing salt for another cation on a suitable ion exchange resin.

Compounds of formula I may be prepared by several routes using methods known in the art.

For example, compounds of formula I can be prepared by condensing an acid of formula IV, or a reactive derivative thereof, with an amino acid of the formula V, or a reactive derivative thereof:

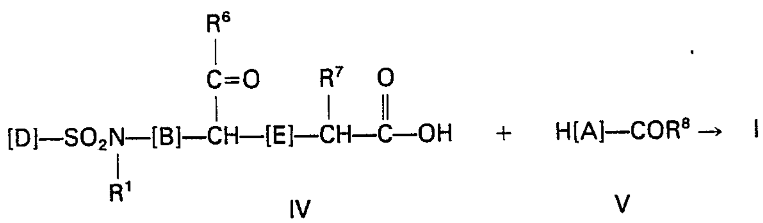

IV V

wherein $R^1$, $R^6$, $R^7$, $R^8$, D, B, E and A are as defined for formula I. The reaction may be carried out in an inert solvent such as dimethylformamide (DMF) in the presence a condensing agent such as 1-(3-dimethylaminopropyl)-3-ethyl carbodiimide hydrochloride (DEC) and 1-hydroxybenzotriazole, and where the compound of formula IV is in the form of a salt in the presence of a base such as triethylamine. The reaction is preferably carried out in an inert atmosphere at a temperature of 0—25°C.

Compounds of formula V are known in the art, or may be prepared by methods well known to those skilled in the art.

Compounds of formula IV may be prepared for example from the reaction of a sulfonyl chloride of formula VI and an amine of formula VII:

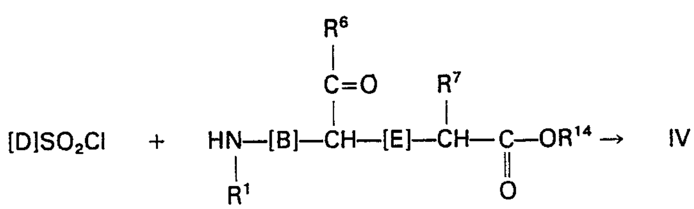

VI VII

wherein D, B, E, $R^1$, $R^6$ and $R^7$ are as defined above and $R^{14}$ is a readily removable ester protecting group such as t-butyl, benzyl or trimethylsilylethyl. The reaction is carried out at 0—5°C in a solvent such as tetrahydrofuran (THF).

Compounds of formula VI may be prepared from known starting materials using procedures well known in the art. For example, when D is of the formula IIIa wherein G is $SO_2$ and $R^3$ is phenylethyl, the sulfonyl chlorides of formula VI may be obtained by reacting a disulfonyl chloride of formula VIII with

aqueous ammonia at low temperature (dry-ice-acetone bath) in a solvent such as 1,2-dimethoxyethane (DME) in the presence of a base such as triethylamine to obtain a sulfonamide of formula IX, followed by reaction of the sulfonamide with phenyl propanal in a solvent such as DME and in the presence of an acid such as p-toluenesulfonic acid:

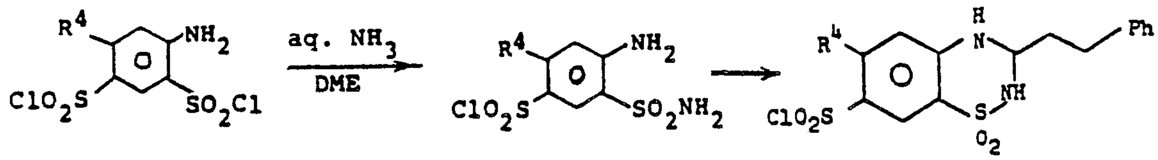


VIII IX VI

(wherein $R^2$=H, $R^3$=phenylethyl, G = —$SO_2$—)

Similarly, when D is of formula IIIb, the sulfonyl chlorides of formula VI may be obtained by well known procedures. A typical reaction scheme was follows:

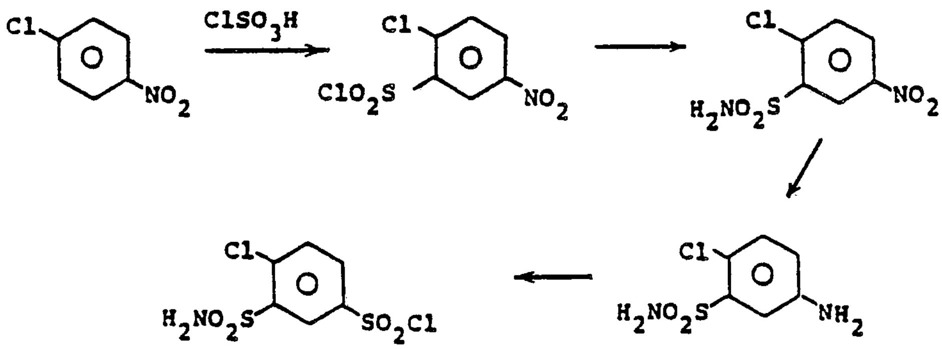


Compounds of formula VII are prepared from known starting materials using methods known in the art. A typical reaction scheme is shown below for compounds of formula VIIb, wherein $R^1$ is hydrogen, $R^6$ is $C_1$—$C_6$-alkyl, $R^7$ is methyl, J is —($CH_2$)—, L is phenyl, M is —$CH_2$—O—$CH_2$—, E is —NH—, and $R^{14}$ is as defined above:

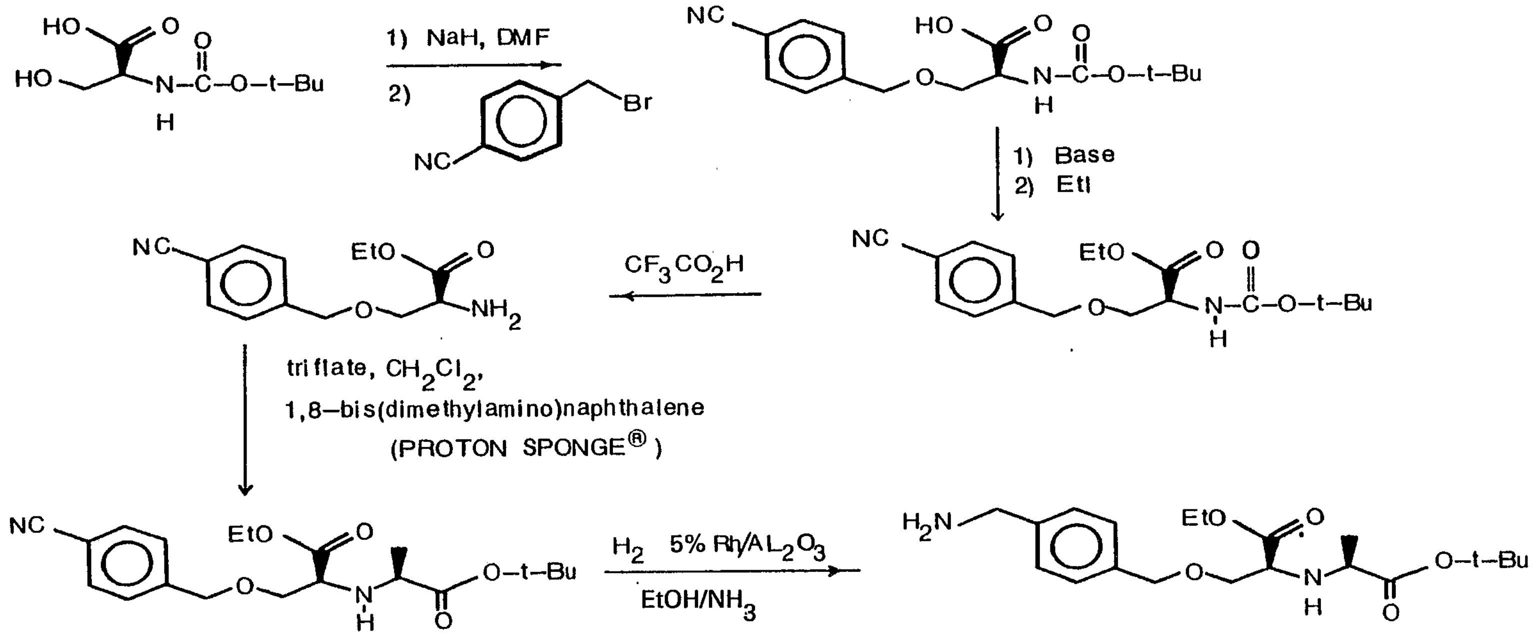


VIIa VIIb

Details of the above typical reaction scheme are disclosed in Example 1, Parts A—E.

In the above reaction scheme, the triflate reagent, i.e. t-butyl 2(S)-(trifluoromethane-sulfonyloxy)propionate, reacts by nucleophillic displacement with the α-amino acid ester to give a high yield of the corresponding specific diastereomer of the resulting monoamino dicarboxylic acid ester.

Compounds of formula VIId, wherein M is $(CH_2)_2$ and $R^1$, $R^6$, $R^7$, $R^{14}$, J, L and E are as described above for formula VIIb, may be prepared as follows:

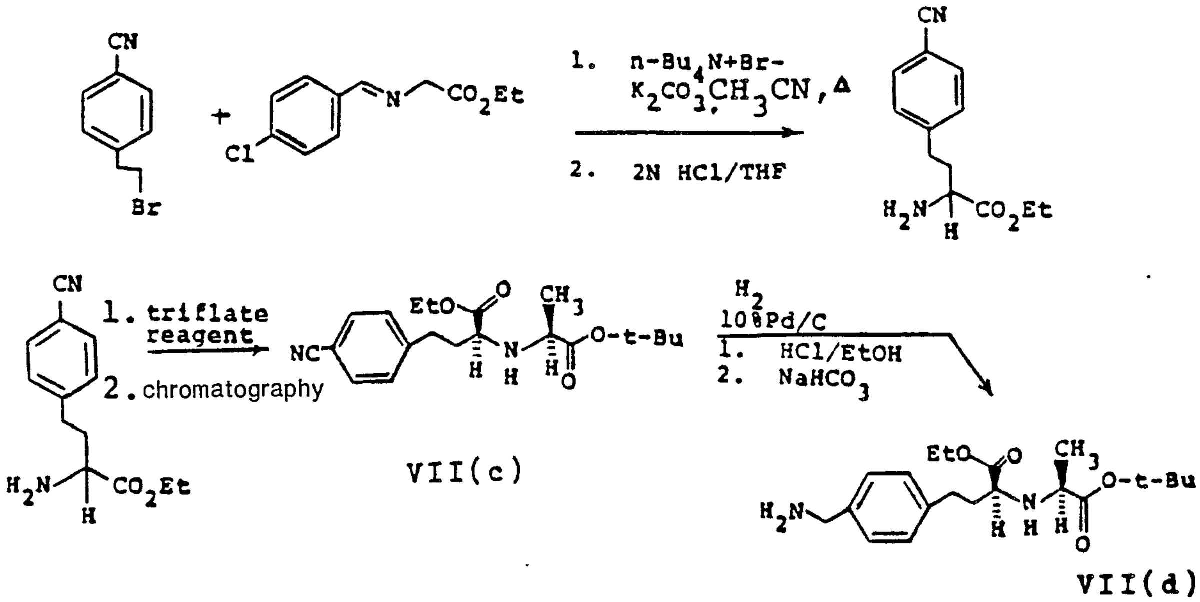

VII(c)
VII(d)

The above reaction scheme is exemplified in Parts A—C of Example 3.

A typical reaction scheme for the preparation of compounds of formula VIIf wherein L is a bond, J is $-(CH_2)_s-$ and M is $-(CH_2)_u-$ wherein the sum of s and u is 4, and $R^1$, $R^6$, $R^7$, $R^{14}$ and E are as described above for formula VIIb is as follows:

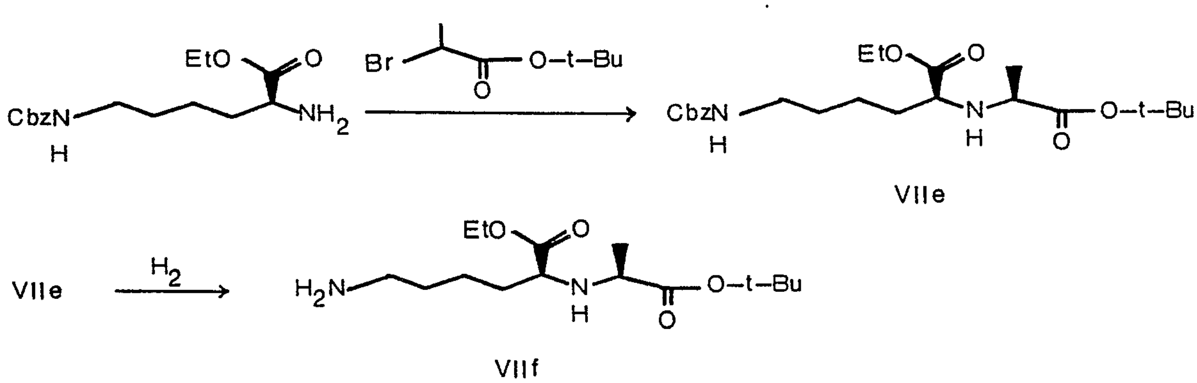

VIIe
VIIf

Example 5 provides details of this procedure.

Alternatively, intermediates of formulae VIIa or VIIc may be used to prepare compounds of formula I as follows: The $R^{14}$ protecting groups (e.g. t-butoxycarbonyl) of compounds of formulae VIIa or VIIc may be removed, e.g. by trifluoroacetic acid, and the nitrile reacted with an amino acid derivative of formula V under the conditions described on page 7. The resulting nitrile can then be reduced to the corresponding amine, e.g., by hydrogenation, which amine can then in turn be coupled to a sulfonyl chloride of formula VI by conventional methods. Similarly, intermediates of formula VIIe (i.e., compounds of formula VII wherein B is alkyl) may be deprotected at the carboxylic acid group and condensed with the amino acid derivative of formula V as described above, then deprotected at the amino group, e.g. by hydrogenation, and the resultant amine reacted with a sulfonyl chloride of formula VI by conventional methods.

Another method for preparing compounds of formula IV is that exemplified below for preparing compounds wherein D is of formula IIIa wherein G is $-SO_2-$, J is $-CH_2-$, L is -aryl-Z— wherein aryl is phenyl and Z is

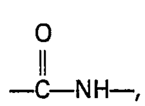

M is $-CH_2-$, E is —NH—, $R^1$ is hydrogen, $R^6$ is ethyl, $R^7$ is methyl, and $R^2$, $R^3$ and $R^4$ are as defined above:

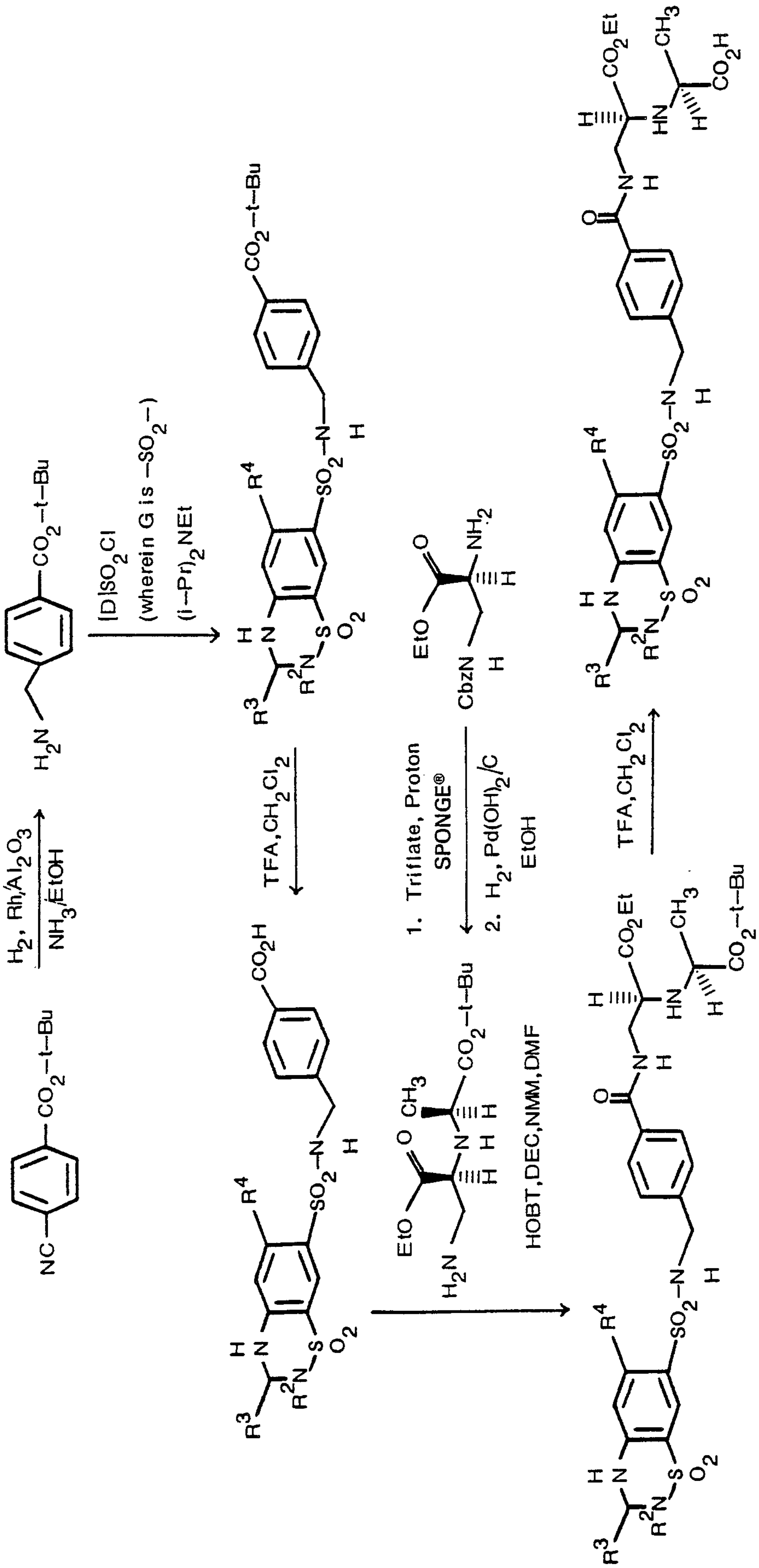


Example 7 incorporates the above procedure.

Another method for the preparation of compounds of formula I comprises condensing an amine of the formula X:

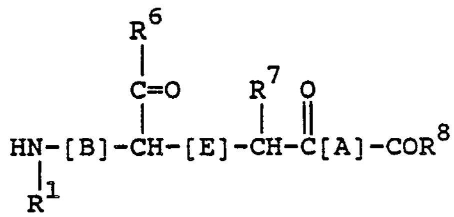

(X)

with a sulfonic acid of the formula VIa: $[D]SO_2OH$, or a reactive derivative thereof, such as the acid chloride, wherein $R^1$, $R^6$, $R^7$, $R^8$, B, D, E and A are as defined above. The reaction may be carried out in an inert solvent such as tetrahydrofuran in the presence of a proton acceptor such as N-methylmorpholine. The reaction is preferably carried out in an inert atmosphere at a temperature of 0—25°C.

Compounds of formula X may be prepared by well known methods, for example to obtain compounds wherein B is $—CH_2—C_6H_5—(CH_2)_2—$, E is —NH— and $R^3$, $R^6$, $R^7$ and $R^8$ are as defined above, compounds of formula VII(c) may be deprotected at the terminal carboxy group and then condensed with an amino acid of formula V:

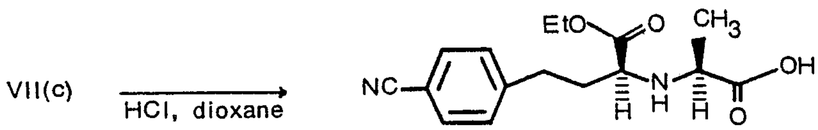


XI

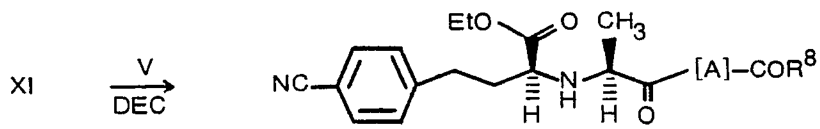


XII

The nitrile of formula XII is reduced to an amine of formula X, e.g. by hydrogenation.

Compounds of formula I may also be prepared by condensing an aldehyde of formula XIII (or a reactive derivative thereof, e.g., an acetal) with an aminosulfonamide of formula XIV:

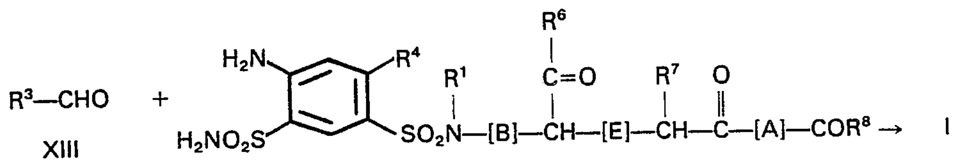


XIV

wherein $R^1$, $R^3$, $R^4$, $R^6$, $R^7$, $R^8$, B, E and A are as defined above. The reaction is carried out in an inert solvent such as tetrahydrofuran in the presence of p-toluenesulfonic acid. The reaction is preferably carried out in an inert atmosphere at a temperature of 0—80°C.

Compounds of formula XIII are known in the art or may be prepared by known methods.

Compounds of formula XIV can also be prepared by known methods, for example a sulfonamide of formula IX can be reacted with an amine of formula VII to obtain a compound of formula XV:

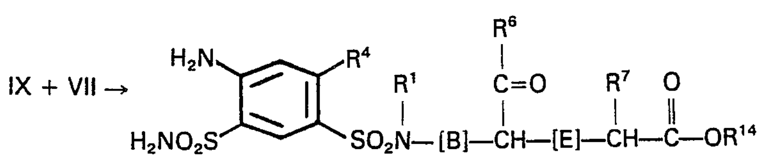

XV

wherein $R^1$, $R^4$, $R^6$, $R^7$, $R^{14}$, B and E are as defined above. The protecting group $R^{14}$ is then removed e.g., by treating with hydrochloric acid in a solvent such as dioxane. The resultant product is condensed with an amino acid of formula V under conditions similar to those described above for the reaction of compounds of formulae IV and V to obtain a compound of formula XIV.

Yet another process for the preparation of the compounds of the formula I wherein X is sulfur comprises condensing a halide of the general formula XV

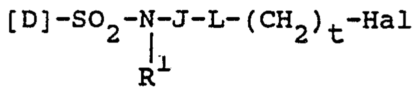

(XV),

with a thiol of the general formula XVI

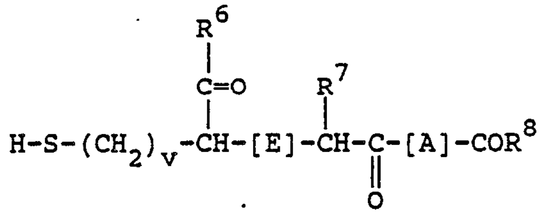

(XVI),

where $R^1$, $R^6$, $R^7$, $R^8$, A, E, J, L, t and v are as defined for formula I and Hal represents halogen, preferably bromine. The reaction is preferably carried out in an inert medium at a temperature of 0—25°C.

Compounds of the formula XV may be prepared by well known methods. Illustrative of such methods, is the following specific reaction scheme:

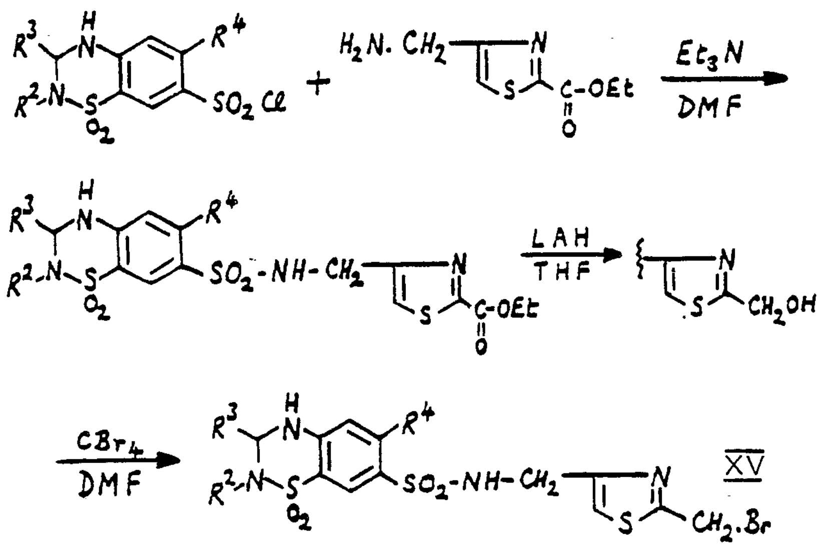

Compounds of general formula XVI may also be prepared by known methods, the following specific reaction scheme being illustrative of such methods:

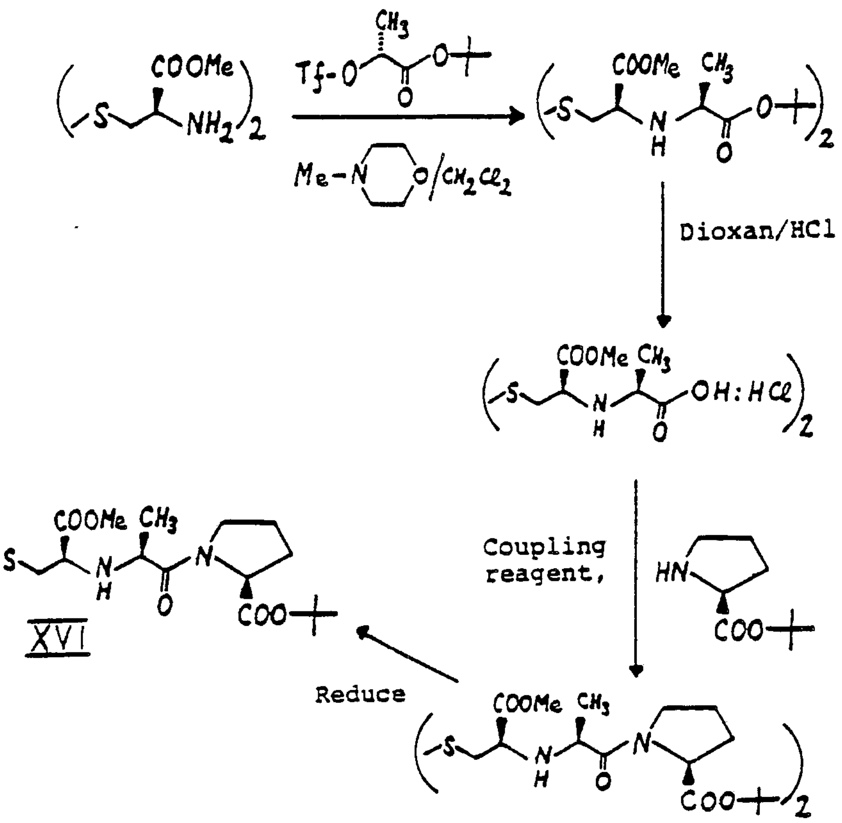


XVI

The known coupling methods above include amino group protection during the coupling reaction, for example with protecting groups such as N-formyl, N-t-butoxycarbonyl (t-Boc) and N-carbobenzyloxy (Cbz) groups, followed by their removal to yield compounds of formula I. Furthermore, the $COR^8$ function wherein $R^8$ is OH may be protected by removable ester groups such as benzyl, ethyl, t-butyl, trimethylsilylethyl and the like.

The more complex esters at $R^6$ (i.e., $R^6$ is other than hydroxy or alkoxy) are most conveniently prepared by esterifying compounds of formula I wherein $R^6$ is hydroxy and $R^8$ is a protected hydroxy, e.g. benzyloxy, with the appropriate reagents, e.g. chloromethyl pivalate in the presence of base, to obtain the corresponding pivaloyloxymethyl ester; the benzyl group is then removed by conventional means, e.g. catalytic hydrogenation.

The following procedures and examples further illustrate the preparation of compounds of this invention.

Preparation 1
t-butyl 2(S)-(trifluoromethanesulfonyloxy)propionate
(Triflate Reagent)

A. Add 2(S)-(p-toluenesulfonyloxy)propionic acid (4.4 g) to a cold solution of 10 ml of isobutylene and 0.4 ml of concentrated sulfuric acid in 30 ml of methylene chloride in a pressure vessel, seal, and agitate at room temperature for 48 hours. Pour into 50 ml of 15% sodium carbonate solution, dry over magnesium sulfate and concentrate to obtain t-butyl 2(*S*)-(p-toluenesulfonyloxy)propionate as an oil (NMR δ 1.37). Distilled material (Kugelrohr, 120°) has $(\alpha]_D^{26}$ = −45.9° (EtOH, c=1).

B. Combine the product of part A (100 g) with acetic acid (40.0 g) and triethylamine (67.2 g) in 200 ml of dry DMF. Heat at 65° for 20 hours. Partition with 21 each ether and water, and wash with ether with citric acid, then with dilute sodium bicarbonate solution. Dry and concentrate the ether solution to obtain t-butyl 2(*R*)-acetoxypropionate as a colorless liquid, bp 50°C/0.1 mm.

C. Combine the product of part B (62.6 g) with ethylenediamine (11.6 g) and heat at 70° for 24 hours, allow to cool, add 300 ml ether and filter. Wash the ether with water, 10% citric acid, and then with sodium bicarbonate solution. Dry and concentrate the ether solution to leave a colorless oil. Crystallize from hexane at −20° to give t-butyl 2(*R*])-hydroxypropionate as white needles, m.p. 41—2°C.

D. Combine the product of part C (10 g) with pyridine (6 ml) in 100 ml methylene chloride. Cool to −70°C, and add dropwise over a period of about 45 minutes a solution of trifluoromethanesulfonic anhydride (13.5 g) in 20 ml methylene chloride, maintaining the temperature below −15°C. Stir at −20°C for

30 min. Add ether and wash successively with water, 4 of aq HCl, sat'd $NaHCO_3$ and brine. Dry and concentrate the organic layer to obtain the title compound.

Preparation 2

6-chloro-3,4-dihydro-3-(2-phenylethyl)-2H-1,2,4-benzothiadiazine-7-sulfonyl chloride 1,1-dioxide

A. Dissolve 5 g 2-chloroaniline-3,5-disulphonyl chloride in 20 ml DME, cool to in a dry-ice/acetone both and add 2 ml triethylamine.

Add dropwise 25% ammonium hydroxide in water (1 ml) in DME (4 ml), stir at in a dry-ice acetone bath for 1 hour, allow to warm to room temperature, and stir for 90 min. Dilute the resultant reaction mixture with ethyl acetate, wash with 4% aq HCl, water and brine, dry over $MgSO_4$ and evaporate to obtain a solid residue.

B. Combine 13.6 g of the sulfonamide prepared in Step A, 6.57 g phenyl propanal, 25 ml DME and 20 mg p-toluene-sulfonic acid and stir at room temperature under $N_2$ for 3 hours. Evaporate the solvent, dissolve the resultant residue in 250 ml ethyl acetate, wash with 100 ml sat'd aq. $NaHCO_3$, and 100 ml brine, then dry over $MgSO_4$, filter and evaporate the solvent to obtain the crude title compound. Purify the crude residue by precipitation in $CH_2Cl_2$; mp 167.0—167.5°C.

Preparation 3

Cis,syn-octahydroindole-2(S)-carboxylic acid, t-butyl ester

A. Dissolve the product of Preparation 4 (77 g) in absolute ethanol (900 ml), add 5% Pd/C (10 g) and hydrogenate at room temperature at an initial pressure of 60 p.s.i. After 3 hours, filter off the catalyst and wash with hot methanol. Evaporate the combined filtrate and wash *in vacuo*, triturate the resultant residue in ethanol (100 ml), chill the solution, then filter and air dry the resultant precipitate to obtain a residue, m.p. 269—270°C.

B. Suspend the product of Part A in dioxane (400 ml) and conc. $H_2SO_4$ (40 ml), add isobutylene (300 ml) and shake in a Parr shaker for 28 hours. Pour the resultant reaction mixture into 50% aqueous NaOH (150 ml) in 500 ml ice water and extract with ethyl ether (3 × 500 ml). Wash the combined organic extracts with water, then brine. Dry the organic layer over $Na_2SO_4$ and evaporate the solvent to obtain the title compound.

Preparation 4

Cis,syn-octahydroindole-2(S)-carboxylic acid benzyl ester

A. Dissolve 27.0 g of ethyl indole-2-carboxylate in 250 ml of trifluoroacetic acid. Add 2.05 g of platinium oxide, hydrogenate the mixture at 50 $lb/in^2$ at room temperature. Filter the mixture and concentrate the filtrate *in vacuo* to give a residue. Suspend the residue in ether and treat with cold dilute sodium hydroxide solution. Dry the organic layer over magnesium sulfate and concentrate it to give ethyl octahydroindole-2-carboxylate, a pale yellow oil.

B. Dissolve 116 g 10-d-camphorsulfonic acid in 1 liter of warm ethyl acetate and add a solution of 86 g of the product of part A in 1 liter of ethyl acetate. Allow the mixture to crystallize heat to reflux, cool to room temperature, and filter. Recrystallize the filter cake from a mixture of 500 ml isopropanol and 1800 ml ethyl acetate, filter and dry the crystals to obtain 2-(S)-carboethoxy-*cis,syn*-octahydroindole, d-10-camphorsulfonate, m.p. 192—193°C.

C. Heat the product of Part B (107.6 g) and d-10-camphor-sulfonic acid (6.35 g) in benzyl alcohol (270 ml) at 105°C under vacuum for 6 hours or until TLC (silica, elute neutralize sample with ethyl ether) indicates reaction is complete. Pour the resultant residue into ethyl ether, seed and stir to obtain a precipitate. Filter the precipitate, wash with ethyl ether (2 × 500 ml) and dry the resultant residue under vacuum to obtain 2-(S)-benzyloxy-*cis,syn*-octahydro-indole, d-10-camphorsulfonate, m.p. 114—118°C.

D. Suspend the product of Part C (150 g) in ethyl ether (1500 ml), add 1N aqueous NaOH (300 ml) and stir until the solid dissolves. Separate the organic layer and wash the aqueous layer with ethyl ether (2 × 200 ml). Combine the organic layer, wash with brine, dry over $Na_2SO_4$ and evaporate the solvent to obtain the title compound.

Preparation 5

6-chloro-3,4-dihydro-3-(chloromethyl)-2*H*-1,2,4-benzothiadiazine-7-sulfonyl chloride 1,1-dioxide

A. Dissolve the sulfonamide prepared in Part A of Preparation 2 (20 g) in dry DME (100 ml), add chloroacetaldehyde dimethyl acetal (10 ml) and p-toluenesulfonic acid and reflux for 3 hours or until TLC (silica, 3% ethyl acetate in $CH_2Cl_2$) indicates reaction is complete. Evaporate the solvent, dissolve the resultant residue in ethyl acetate, wash with saturated $NaHCO_3$, then brine and concentrate to half volume. Refrigerate overnight, filter the resultant precipitate, wash in hexane, filter and dry to obtain the title compound.

Example 1

1-[2-(S)-[[1-(S)-carboethoxy-2-[4-[[[6-chloro-3,4-dihydro-3-(2-phenylethyl)-2H-1,2,4-benzothiadiazin-7-yl]sulfonylamino]methyl]phenylmethoxy]ethyl]amino]-1-oxopropyl)-[2S-[2α, 3aα, 7aα)]-octahydro-1H-indole-2-carboxylic acid, S,S-dioxide

A. to 10.4 g NaH (50% in mineral oil, washed with hexane) in 50 ml DMF at 0—5°C, add dropwise over a 1 hour period 20 g of N-a-t-butoxycarbonyl-L-serine in 350 ml DMF. Stir at room temperature for 1 hour, then at 45°for 1 hour. Cool the reaction mixture to 0—5°C and add dropwise over 30 minutes 21.3 g of p-cyanobenzylbromine in 100 ml DMF. Stir at 0°C for 80 minutes, add 30 ml water, stir and filter. Concentrate the filter and partition between ethyl acetate and sat'd aq. $NaHCO_3/H_2O$. Wash the aqueous phase with ethyl acetate, adjust to pH 7.5 with 6N HCl and concentrate to approximately 100 mls.

B. To the product of Step A, add 60 ml methanol, 40 ml ethyliodide, and 4 g $NaHCO_3$. Stir under a nitrogen atmosphere for 72 hours, evaporate the solvent *in vacuo* and partition the residue between 800 ml ethyl acetate and 800 ml water. Separate the organic layer, and extract the aqueous layer with ethyl acetate; combine the organic extracts, wash with brine, dry over $MgSO_4$, filter and evaporate the solvent. Purify the resultant residue by High Pressure Liquid Chromatography (HPLC) using 2 Prep 500 cartidges and eluting with 21% ethyl acetate in hexane. Combine the desired fractions and evaporate the solvent to obtain a residue. FAB mass spec m/e = 349 (M+H).

C. Cool 2 g of the product of Step B to 0—5°C and add dropwise 25 ml trifluoroacetic acid. Let stand until TLC (silica, elute with hexane:ethyl acetate) indicates no starting material is left. Add ethyl acetate, then evaporate the solvent *in vacuo*. Dissolve the resultant residue in ethyl ether, and wash with 1N aqueous NaOH; backwash the aqueous phase with ether, combine the ethereal extracts, dry over $K_2CO_3$ and evaporate the solvent to obtain a residue.

D. Cool 1.6 g triflate reagent (Preparation 1) in 10 ml $CH_2Cl_2$ to 0°C. Add dropwise 1.1 g of the product of Step C and 1.2 g of PROTON SPONGE® (1,8-bis(dimethylamino)naphthalene, Aldrich Chemical Co., Milwaukee, WI) in 20 ml $CH_2Cl_2$. Monitor reaction by TLC, adding 1,8-bis(dimethylamino)naphthalene and triflate reagent as necessary. Filter the resultant precipitate with the aid of ethyl acetate, evaporate the solvent, and purify the resultant residue by column chromatography, eluting with 30% ethyl acetate in hexane. FAB mass spec m/e = 377 (M+H).

E. Dissolve 2.5 g of the product of Step D in 50 ml ethanol saturated with $NH_3$, add 1.25 g 5% $Rh/Al_2O_3$ and hydrogenate at 60 psi for 4 hours. Filter the resultant mixture through celite with the aid of ethanol, then evaporate the solvent *in vacuo* to obtain a residue.

F. Cool to 0°C 2.5 g of the product of Step E in 25 ml dry THF and add dropwise 3.3 g 6-chloro-3,4-dihydro-3-(2-phenylethyl)-2H-1,2,4-benzothiadiazine-7-sulfonyl chloride 1,1-dioxide in 20 ml dry THF. Stir at 0°C for 1 hour, then add 0.6 ml N,N-diisopropylethylamine and stir at room temperature for 2 hours. (Reaction may be monitored by TLC [silica; elute with hexane: ethyl acetate]). Add the reaction mixture to ethyl acetate, wash with 4% aq. HCl, sat'd $NaHCO_3$ and brine, then dry over $MgSO_4$ and evaporate the solvent *in vacuo.* Purify the resultant residue by HPLC: dissolve the residue in acetone:ethyl acetate:hexane (20:35:45) and separate on 2 Prep 500 cartridges using acetone:ethyl acetate:hexane (5.5:36.5:58) as mobile phase. Monitor eluent by TLC (silica; elute with acetone:ethyl acetate:hexane (6:39:55]), combine the desired fractions and evaporate the solvent *in vacuo* to obtain a residue. FAB mass spec m/e = 766 (M+H).

G. Stir 2.9 g of the t-butyl ester of Step F in 40 ml HCl/dioxane overnight; pass nitrogen through the solution to evaporate the solvent and obtain the free acid.

H. Dissolve 2.9 g of the product of Step G in 6 ml DMF, add 1.1 g cis,syn-octahydro-1H-indole-2(S)-carboxylic acid, t-butyl ester and 700 mg 1-hydroxybenzotriazole. Cool to 0°C, add 0.7 ml triethylamine and 900 mg 1-(3-dimethylaminopropyl)-3-ethyl carbodiimide hydrochloride and stir overnight. Evaporate the solvent in vacuo, take up the residue in ethyl acetate, and wash with water, 4% aq. HCl, sat'd aq. $NaHCO_3$, and brine. Dry the organic layer over $MgSO_4$, filter and evaporate to obtain a residue.

Purify said residue by HPLC using 2 Prep 500 cartridges and methanol:ethyl acetate:hexane (5.25:36:58.75) as mobile phase (residue dissolved in methanol:ethyl acetate:hexane [10:35:55]). Combine the desired fractions as determined by TLC and evaporate the solvent. Rechromatograph the resultant residue by HPLC using acetone:ethylacetate:hexane (10:40:50) as mobile phase, combining the desired fractions and evaporating the solvent to yield the title compound as a t-butyl ester. FAB mass spec m/e = 917 (M+H).

I. Stir 1.5 g of the product of Step H in 20 ml dioxane saturated with HCl overnight; pass nitrogen through the solution to evaporate the solvent. Purify the resultant residue on Dowex A 50 2X ($H^+$ form), eluting with (ethanol: water (1:1]):pyridine (95:5). Combine desired fractions as determined by TLC (silica; $CH_2Cl_2$:MeOH:AcOH [90:5:3]) and evaporate the solvent to obtain the title compound. FAB mass spec m/e = 861 (M+H).

Example 2

1-[2-(S)-[[1-(S)-carboxy-2-[4-[[[6-chloro-3,4-dihydro-3-(2-phenylethyl)-2H-1,2,4-benzothiadiazin-7-yl]sulfonylamino]methyl]phenylmethoxy]ethyl]amino]-1-oxopropyl]-[2S-(2α, 3aα, 7aα)]-octahydro-1*H*-indole-2-carboxylic acid, *S,S*-dioxide.

A. Treat the product of Example 1, Step G in a manner similar to that described in Example 1, Step H, first paragraph, substituting cis,syn-octahydro-1H-indole-2(S)-carboxylic acid, benzyl ester camphorsulfonate salt (see Preparation 4, Part C) for the t-butyl ester.

Purify the resultant residue by column chromatography on 100 g silica eluted with $CHCl_3$:ethyl acetate. Combine the desired fractions as determined by TLC (silica; elute with $CH_2Cl_2$:methanol:acetone (93:2:5]) and evaporate the solvent to obtain a residue. Further purify the residue on a sephadex column (350 g). FAB mass spec m/e = 951 (M+H).

B. Suspend 450 mg of the diester obtained in Step A in 2 ml water. Add 2 ml 1N aq. NaOH and stir

overnight at room temperature. Adjust to pH 6—7 with 1N HCl, filter the resultant solid and dry under vacuum to obtain the title compound. FAB mass spec m/e = 833 (M+H).

Example 3

1-[2-(S)-[[3-[4-[[[6-chloro-3,4-dihydro-3-(2-phenylethyl-2*H*-1,2,4-benzothiadiazin-7-yl]sulfonylaminomethyl]phenyl]-1-(S)-(ethoxycarbonyl)propyl]amino]-1-oxopropyl]-[2S-(2α, 3aα, 7aα]-octahydro-1*H*-indole-2-carboxylic acid, S,S-dioxide hydrochloride

A. 2-Amino-4-(4-cyano)phenylbutanoic acid, ethyl ester

Reflux 2-(4-cyano)phenylethyl bromide (23 gm), the p-chlorobenzaldimine of ethyl glycinate (21 gm), tetra-n-butylammonium bromide (10 gms) and freshly ground fine potassium carbonate powder (42 gms) in acetonitrile (150 mls) with mechanical stirring under nitrogen for 12 hours.

Cool the mixture, filter off the solid and wash the filter cake with ethyl acetate (3 × 150 mls). Wash the combined filtrate with water (2 × 100 mls) and evaporate the solvent *in vacuo.* Stir the residue vigorously with THF (200 mls) and 2N HCl (200 mls) at room temperature for 2 hours. Wash the aqueous phase with ethyl acetate, basify with solid potassium carbonate to pH 9 and extract with ethyl acetate to give the title compound of Part A.

B. N-[1-(S)-(ethoxycarbonyl)-3-(4-cyano)phenyl]-(S)-alanine, t-butyl ester

Slowly add the product of Part A (9 gms) and PROTON SPONGE® (17.2 gm) in dry dichloromethane (80 mls) dropwise into a stirred solution of triflate reagent (22 gm) in dry dichloromethane (40 mls) cooled in an acetone-ice bath. Stir at room temperature overnight. Filter the resultant precipitate and wash the filter cake with ethyl acetate (5 × 100 ml). Wash the combined filtrate with 10% citric acid (3 × 100 ml); sodium bicarbonate (sat'd, 2 × 100 ml), and saturated brine (2 × 100 ml). Dry the solution over potassium carbonate in the presence of triethyl amine (5 mls), and remove the solvent *in vacuo.* Chromatograph the resultant residue (hexane:EtOAc:$CH_2Cl_2$ [8:1:1], 1% $Et_3N$; 500 gm silica gel; 230—400 mesh) to obtain the title compound of Part B.

C. N-[3-(4-aminomethyl)phenyl-1-(S)-(ethoxycarbonyl)propyl]-(S)-alanine, t-butyl ester

Hydrogenate a mixture of the product of Part B (2 g), hydrogen chloride (0.2 gm) and 10% Pd/C (0.4 gm) in absolute ethanol (100 ml) at 50 psi for 5 hours. Filter the resultant mixture through celite. Evaporate the solvent to obtain the hydrogen chloride salt of the title compound of Part C.

D. Add N-methylmorpholine (0.5 ml) to a solution of the product of Part C (1 gm) in dry THF (20 mls) cooled in acetone-ice bath (−5°C). Add the sulfonyl chloride of Preparation 2 (1.3 gm) and stir the resulting mixture at room temperature overnight. Dilute the resultant reaction mixture with ethyl acetate (400 ml), wash with 0.5N HCl (100 ml), saturated $NaHCO_3$ (2 × 100 ml), and brine (2 × 100 ml). Dry the solution over $MgSO_4$ and remove the solvent *in vacuo.* Purify the resultant residue by chromatography [400 gm silica gel, 230—400 mesh; first hexane:EtOAc:$CH_2Cl_2$, 4:1:1, then hexane:EtOAc:$CH_2Cl_2$, 1:1:1] to obtain a residue.

E. Add the product of Part D (1.76 gm) to 5.5 M HCl in dioxane (50 ml) and stir the resulting mixture at room temperature overnight. Evaporate the solvent *in vacuo,* triturate the solid residue with ether, and remove the solvent *in vacuo* to obtain a residue (hydrogen chloride salt).

F. Treat the product of part E in a manner similar to that described in Example 1, Part H, first paragraph, substituting N-methylmorpholine for triethylamine to obtain a residue. Purify the resultant residue by chromatography [400 gm silica gel 230—400 mesh; hexane:EtOAc:$CH_2Cl_2$, 1:2:1] to obtain the t-butyl ester of the title compound.

G. Stir the product of Part F (0.9 gm) in 5.5 M HCl/dioxane (40 mls) at room temperature for 2 hours. Remove the solvent *in vacuo,* triturate the product with ether, and dry *in vacuo* to obtain the title compound of Example 3. FAB mass spec m/e=845 (M+H).

Example 4

1-[2-(S)-[[1-(S)-carboxy-3-[4-[[[6-chloro-3,4-dihydro-3-(2-phenylethyl)-2*H*-1,2,4-benzothiadiazin-7-yl]sulfonylamino]methyl]phenyl]-propyl]amino-1-oxopropyl]-[2S-(2α, 3aα, 7aα)]-octahydro-1*H*-indole-2-carboxylic acid, *S,S*-dioxide

Dissolve the product of Example 3 (0.85 gm) in methanol (2 mls) and cool to 0°C under nitrogen. Add 1N sodium hydroxide (5 mls) portionwise. Refrigerate the mixture overnight. Acidify the resultant mixture with acetic acid, evaporate to dryness, and purify the residue by chromatography on a C—18 medium pressure reverse-phase column to yield the title compound of Example 4.

Example 5

1-[[2-(S)-[5-[[6-chloro-3-chloromethyl-3,4-dihydro-2*H*-1,2,4-benzothiadiazin-7-yl]sulfonylamino]-1-(S)-(ethoxycarbonyl)pentyl]amino]-1-oxopropyl]-(2S-(2α, 3aα, 7aα)]-octahydro-1*h*-indole-2-carboxylic acid, S,S-dioxide

A. Combine $N^{\varepsilon}$Cbz lysine, ethyl ester (30.0 g), t-butyl bromopropionate (44.78 g), triethylamine (14.8 ml) and DMF (120 ml) and stir under nitrogen at 75°C until TLC shows no starting material present. Evaporate the solvent, dilute the resultant residue with water and extract with ethyl ether. Wash the organic layer with brine, dry over $MgSO_4$, filter and evaporate the solvent *in vacuo.*

Purify the resultant residue by column chromatography on 1,100 g silica gel (60—200 mesh), eluting with ether:hexane (50:50→75:50). Combine the desired fractions and evaporate the solvent in air. Further purify the resultant residue by HPLC using 2 Prep 500 cartridges and eluting with ether:hexane. Combine desired fractions, dry over $MgSO_4$ and evaporate the solvent in air to obtain a residue.

B. Combine 20% $Pd(OH)_2/C$ (21.1 g) and anhydrous ethanol (25 ml) in a Parr Shaker bottle, add the product of Part A (16.5 g) in ethanol (55 ml) and hydrogenate overnight under 60 p.s.i. $H_2$. Filter the resultant solution over filter paper and celite and evaporate the solvent to obtain a residue.

C. Dissolve the sulfonyl chloride prepared in Preparation 5 (2.4 g) in dry DME (15 ml). Add triethylamine (1 ml) and the product of Part B of this Example (2 g) in DMF (10 ml) and stir for 1 hour, or until TLC (silica, 10% MeOH in $CH_2Cl_2$) indicates no starting material is left. Add the resultant solution to ethyl acetate, wash with water, saturated $NaHCO_3$ and brine, dry over $MgSO_4$ and evaporate the solvent *in vacuo*. Purify the resultant residue on a Sephadex LH20 column to obtin a residue. FAB mass spec m/e = 632 (M+H).

D. Treat the product of Part C in a manner similar to that described in Example 1, Part G.

E. Treat the product of Part D in a manner similar to that described in Example 1, Part H, first paragraph, substituting the camphorsulfonate salt of the benzyl ester of the octahydroindole (see Preparation 4 Part C) for the t-butyl ester.

Purify the resultant residue on a sephadex LH20 column, combine the desired fractions and evaporate the solvent. Dissolve the resultant residue in ethyl acetate, add dioxane saturated with HCl and evaporate the solvent to obtain the benzyl ester of the title compound. FAB mass spec m/e = 817 (M+H).

F. Dissolve the product of Part E (600 mg) in acetic acid saturated with hydrogen bromide (6 ml). After 5 hours, evaporate the solvent and purify the resultant reside on a Sephadex LH20 column. Combine the desired fractions as determined by TLC (silica, $MeOH:CH_2Cl_2$:acetic acid, 10:90:4) and evaporate the solvent to obtain the title compound. FAB mass spec m/e = 726 (M+).

Example 6

1-[[2-(S)-[1-(S)-Carboxy-5-[[6-chloro-3-chloromethyl-3,4-dihydro-2*H*-1,2,4-benzothiadiazin-7-yl]sulfonylamino]pentyl]amino]-1-oxopropyl]-[2S-(2α, 3aα, 7aα)]-octahydro-1*H*-indole-2-carboxylic acid, *S,S*-dioxide

Add the product of Example 5, Part E (benzyl ester) to 1N aqueous NaOH (4 ml) and water (4 ml) and stir overnight. Add 1N HCl (4 ml) and ethanol. Charge the resultant solution to Dowex Ag 50 cation exchange resin by stirring batchwise for 20 minutes (60 ml resin, pre-washed with ethanol:water, 1:4). Prepare a column from the loaded resin, elute the column with ethanol:water (1:4) until the eluate is pH 6, then elute with ethanol/water:pyridine (95:5). Combine the desired fractions as determined by TLC (silica, ethanol:water, 9:1). Further purify the resultant product on a Sephadex LH 20 column. Combine the desired fractions and evaporate the solvent to obtain the title compound. FAB mass spec m/e + 698 (M+H).

Example 7

N-[2-[[4-[[[6-chloro-3,4-dihydro-3-(2-phenylethyl)-2*H*-1,2,4-benzothiadiazin-7-yl]sulfonylamino]methyl] phenyl]carbonyl]amino-1-(S)-(ethoxycarbonyl)ethyl]-(S)-alanyl-(S)-proline, *S,S*-dioxide

A. Dissolve 4-cyanobenzoic acid, t-butyl ester (11.34 g) in ethanol (100 ml) saturated with anhydrous $NH_3$, add 5% $Rh/Al_2O_3$ (120 g) and hydrogenate in a Parr apparatus at 60 p.s.i. at room temperature for $2\frac{3}{4}$ hours. filter the resultant solution through celite and evaporate the solvent to obtain a residue.

B. Dissolve the product of Part A (11.27 g) in dry THF (100 ml) add N,N-diisopropylethyl amine (8.44 g) and cool to 0°C in a ice bath. Add dropwise, slowly and with stirring, the sulfonyl chloride prepared in Preparation 2 (27.51 g) and let stand at 0°C for 35 minutes. Remove the ice bath and stir at room temperature for $2\frac{1}{2}$ hours or until TLC (silica, $CH_2Cl_2$:MeOH, 95:5) shows the reaction to be complete. Evaporate the solvent to obtain a residue.

C. Cool to 0°C a solution of the product of Part B (3.00 g) in $CH_2Cl_2$ (25 ml) and add, slowly and with stirring, trifluoroacetic acid (25 ml). Stir for 30 minutes at 0°C, then at room temperature for $2\frac{1}{4}$ hours or until TLC (as in Part B) shows the reaction to be complete. Evaporate the solvent to obtain a residue.

D. Dissolve triflate reagent as prepared in Preparation 1 (3.31 g) in $CH_2Cl_2$ (75 ml) and cool to 0°C; add, slowly and with stirring a solution of PROTON SPONGE® (4.24 g) and N-βCbz-2,3-diaminopropionate, ethyl ester (2.00 g) in $CH_2Cl_2$ (75 ml). Stir at 0°C for 15 hours. Extract the solution with 10% citric acid (2x), then sat'd. $NaHCO_3$ (2x), dry the organic layer over $MgSO_4$, filter and evaporate the solvent. Purify the resultant residue by flash chromatography, eluting with $CH_2Cl_2$:EtOAc (88:12). Combine the desired fractions and evaporate the solvent. Dissolve the Cbz-diester (0.5 g) in ethanol (25 ml) containing 20% $Pd(OH)_2/C$ (0.15 g) and hydrogenate in a Parr apparatus at 50 p.s.i. at room temperature for 1 hour. Filter the resultant solution through celite and evaporate the filtrate *in vacuo*.

Combine the resultant residue (0.33 g) and the product of Part C (0.59 g) in dry DMF (7 ml), cool to 0°C and add slowly 1-hydroxybenzotriazole (0.18 g) followed by N-methyl morpholine (0.13 g), then by DEC (0.25 g). Stir the mixture for 20 min. at 0°C, then at room temperature for 16 hours or until TLC (silica, ethanol:methanol, 85:15) indicates the reaction to be complete. Dilute the reaction mixture with $CH_2Cl_2$ and extract with saturated $NaHCO_3$, then with 10% aqueous citric acid. Dry the organic layer with $MgSO_4$, filter and evaporate the solvent *in vacuo* to obtain a residue.

E. Dissolve the product of Part D (0.75 g) in $CH_2Cl_2$ (5 ml) and cool the 0°C. Add, slowly and with stirring,

trifluoroacetic acid (5 ml) and stir at 0°C for 30 minutes, then at room temperature for 4 hours or until TLC (silica, $CH_2Cl_2$:MeOH, 90:10) indicates no starting material is left. Evaporate the solvent.

Purify the resultant residue by ion exchange chromatography on Biorad AG50—W—X2 resin (100—200 mesh, hydrogen form) previously equilibrated in ethanol:water. Elute with ethanol:water:pyridine, combine the desired fractions and evaporate the solvent to obtain a residue.

F. Treat the product of Part E in a manner similar to that described in Example 3, Parts F and G, substituting proline, t-butyl ester for the octahydro-1*H*-indole to obtain the title compound. FAB mass spec m/e = 873 (M+).

Example 8

N-[5-[[6-chloro-3,4-dihydro-3-(2-phenylethyl)-2*H*-1,2,4-benzothiadiazin-7-yl]sulfonylamino]-1-(S)-(methoxycarbonyl)pentyn-3-yl]-(S)-alanyl-(S)-proline, *S,S*-dioxide, hydrochloride

A. Titrate a solution of diazomethane in ether into a solution of 1-amino-1-(S)-carboxy-5-(t-butoxycarbonyl amino)-3-pentyne (4 g) in ethanol (200 ml) until a yellow color remains. Evaporate the solvent to obtain 1-amino-1-(s)-methoxycarbonyl-5-(t-butoxycarbonylamino)-3-pentyne.

B. Add the product of Part A (4 g) and PROTON SPONGE® (5 g) in dichloromethane (15 ml) dropwise to a stirred solution of triflate reagent (6.5 g) (See Preparation 1) in dichloromethane (10 ml) at −10°C. Stir the solution and slowly allow to warm to room temperature over 2 h. Filter the reaction mixture through celite, washing thoroughly with ethyl acetate. Wash the combined organic solution with 10% citric acid (3×), saturated sodium bicarbonate (2×) and brine (2×), dry over $MgSO_4$ and evaporate the solvent. Chromatograph on silica gel, eluting with ethyl acetate:hexane (1:4) containing 1% triethylamine to obtain 1-(S)-methoxycarbonyl-5-(t-butoxycarbonylamino)pentyn-3-yl-(S)-alanine, t-butyl ester.

C. Add the product of Part B (3.5 g) to a stirred solution of 4 M HCL in dioxane (25 ml) at 0° and stir for 0.5 hours. Evaporate the solvent and triturate the residue with ether to obtain 1-(S)-methoxycarbonyl-5-amino-pentyn-3-yl-(S)-alanine, t-butyl ester, hydrochloride.

D. Add N-methylmorpholine (1.5 g) to a solution of the product of Part C (2.9 g) in tetrahydrofuran at 0°. Add 6-chloro-3,4-dihydro-2-(phenylethyl)-2H-1,2,4-benzothiadiazine-7-sulfonyl chloride, *S,S*-dioxide (3.9 g) (See Preparation 2) and stir the resulting mixture at room temperature overnight. Dilute the reaction mixture with ethyl acetate, wash with 0.5N HCl (1×), saturated sodium bicarbonate (2×), and brine (1×), dry over $MgSO_4$ and evaporate the solvent. Chromatograph on silica gel, eluting with ethyl acetate:dichloromethane to obtain a residue.

E. Add the product of Part D (4.8 g) to a 4 M solution of HCL in dioxane (100 ml) and stir the resulting mixture at room temperature overnight. Evaporate the solvent then triturate the residue with ether to obtain a residue.

F. Treat the product of Part E in a manner similar to that described in Example 3, Part F, first paragraph, substituting (S)-proline, t-butyl ester for the octahydro-1*H*-indole, to obtain a residue.

Purify the resultant residue by chromatography on silica gel, eluting with ethyl acetate:$CH_2Cl_2$. Combine the desired fractions and evaporate the solvent to obtain a residue.

G. Treat the product of Part F in a manner similar to that described in Example 3, Part G to obtain the title compound.

Example 9

N-[S-[[6-chloro-3,4,-dihydro-3-(2-phenylethyl)-2*H*-1,2,4-benzothiadiazin-7-yl]sulfonylamino]-1-(S)-methoxycarbonyl)-(E)-penten-3-yl]-(S)-alanyl-(S)-proline, *S,S*-dioxide, hydrochloride

A. Add a solution of 1-N-acetylamino-1-(S)-carboxy-5-(t-butoxycarbonylamino)-3-pentyne (5 g) in tetrahydrofuran (20 ml) dropwise to a stirred solution of sodium (1.2 g) in liquid ammonia (1.2 L). After 2 h, add ammonium hydroxide and water. Remove the ammonia, dissolve the residue in ethyl acetate, wash with 0.5N HCl (2×) and brine (3×), dry over $MgSO_4$ and evaporate the solvent. Purify by precipitation from dichloromethane as the DCHA salt of (*E*)-1-N-acetylamino-1-carboxy-5-(*t*-butoxy-carbonylamino)-3-pentene.

B. Add cobalt chloride hexahydrate (50 mg) and Acylase I (aminoacylase from porcine kidney, grade 1, available from Sigma Chemical Co., St. Louis, MO) (100 mg) to a stirred solution of the product of Part A (3.5 g) in 0.1 M, pH 7.5 phosphate buffer (120 ml) at 38°. After 16 hours, remove the protein with activated carbon and filter through celite. Adjust pH of the solution to 2.75 and remove the unreacted isomer by washing with ethyl acetate (3×). Adjust the pH to 6.5 and evaporate the solvent. Remove the salt from the residue by dissolving in ethanol (100 ml) and filtering through celite. Evaporate the solvent to obtain (*E*)-1-amino-1-(*S*)-carboxy-5-(t-butoxycarbonylamino)-3-pentene.

C. Substitute the product of Part B (i.e., the 3-pentene compound) for the 3-pentyne compound in the procedure of Example 8, Parts B to G to obtain the title compound.

Example 10

1-[2-(S)-[[2-[4-[[[6-chloro-3-chloromethyl-3,4-dihydro-2*H*-1,2,4-benzothiadiazin-7-yl]sulfonyl-amino]methyl]phenyl-1-(S)-(ethoxycarbonyl)propyl]amino]-1-oxopropyl]-(2S-(2α,3aα,7aα)]-octahydro-1-*H*-indole-2-carboxylic acid, t-butyl ester, *S,S*-dioxide

A. To 1 g of the compound of Example 3, part B, in THF, add HCl-saturated dioxane (30 ml). Stir the mixture at room temperature for 5 hours under an inert atmosphere. Evaporate the solvent to obtain a dry foam as a hydrochloride salt.

B. Combine 3 g of the product of Part A with 2.5 g of octahydroindole-t-butyl ester (Preparation 3) in the manner described in Example 3, Part F to obtain 1-[2(S)-[[p-cyanophenyl-1(S)-(ethoxycarbonyl)propyl]-amino]-1-oxopropyl]-[2S-(2α,3aα,7aα)]-octahydro-1H-indole-2-carboxylic acid, t-butyl ester. FAB mass spec. M/e = 501 ($M^+$).

C. Hydrogenate 2.5 g of the product of Part B in 150 ml of ethyl alcohol containing 5 ml of saturated HCl/dioxane at 55 psi in the presence of 500 mg of 10% Pd on C for 20 hours. Filter the resultant reaction mixture through celite to obtain 2.6 g of amine.

D. Combine 850 mg of the product of Part C with 600 mg of 6-chloro-3-chloromethyl-3,4-dihydro-2*H*-1,2,4-benzothiadiazin-7-sulfonyl chloride, *S,S*-dioxide in 5 ml of THF containing 0.8 ml of N-methylmorpholine and let stand overnight at room temperature. Dilute with EtOAC, wash with saturated NaCl, and evaporate the solvent. Purify the resultant residue by chromatography on $SiO_2$ with $CH_2Cl_2$/EtOAC as eluant to produce 700 mg of title compound. FAB mass spectrum shows M/e = 844 ($M^+$).

Example 11

N-[N-[4-[4-[[[[6-chloro-3,4-dihydro-3-[2-(2-pyridinyl)ethyl]-2*H*-1,2,4-benzothiadiazin-7-yl]sulfonylamino]methyl]phenyl]-1-(S)-ethoxycarbonyl)propyl]-(S)-alanyl-(S)-proline, t-butyl ester, *S,S*-dioxide

A. To 6 g of the amine of Example 3, Part C, in 25 ml of DME cooled with an ice-bath, add 1.9 ml of N-methylmorpholine. To this mixture add 5 g of 4-amino-2-chloro-5-sulfonamidobenzenesulfonyl chloride in 25 ml of DME. Stir the reaction overnight with cooling under an inert atmosphere. Concentrate the resultant reaction mixture and partition the residue between EtOAc and $H_2O$. Separate the organic layer and evaporate the solvent. Purify the resultant residue by chromatography on $SiO_2$ using EtoAc/Hexanes as eluant to obtain 5 g of an oil FAB mass spectrum M/e = 633 ($M^+$).

B. Treat the product of Part A in a manner similar to that described in Example 10, Part A, to yield a dry foam. FAB mass spectrum M/e = 614 ($M^+$).

C. Combine 1.6 g of the product of Part B with 490 mg L-Proline, t-butyl ester in the present of 9 ml of DMF, 380 mg 1-hydroxybenzotriazole, 1.14 ml N-methylmorpholine and 550 mg DEC. Stir the reaction overnight, then extract and purify the resultant product as described in Example 3, Part F, using EtoAc/Hexanes as eluent to obtain a residue, FAB mass spectrum M/e = 730 $M^+$.

D. In 7 ml THF, combine the amino-sulphonamide obtained in Part C with 360 mg of (2-pyridinyl)propanal and 10 mg of p-toluenesulfonic acid and stir for 24 hours at room temperature in an inert atmosphere. Concentrate the reaction mixture and partition the resultant residue between EtOAc and water. Separate the organic phase, wash with dilute $NaHSO_3$, saturated $NaHCO_3$, saturated NaCl and dry with $MgSO_4$. Evaporate the solvent and chromatograph the residue on $SiO_2$ using EtOAc as eluant to obtain 350 mg of the title compound. Mass spectrum, M/e = 899 ($M^+$).

Example 12

1-[N-[2-[4-[[[6-chloro-3,4-dihydro-3-(2-phenylethyl)-2H-1,2,4-benzothiadiazin-7-yl]sulfonyl]aminomethyl]thiazol-2-ylmethylthio]-1(S)-(methoxycarbonyl)ethyl]-(S)-alanyl]-cis,syn-octahydroindole-2(S)-carboxylic acid, S,S-dioxide

A) *Ethyl-4-Aminomethylthiazole-2-Carboxylate*

1. Ethyl-thiazole-4-Carboxylate

Combine ethylbromopyruvate (19.5 g, 100 mmole) in 50 ml EtOH and thioformamide (4.27 g, 70 mmole) in 10 ml EtOH and stir at room temperature overnight. Pour into 1N HCl (100 ml) and extract with 190 ml diethyl ether. Separate the aqueous layer and treat with an excess of solid sodium bicarbonate. Extract the aqueous layer with diethylether (2 × 150 ml), dry over $MgSO_4$ and evaporate the solvent to obtain the title compound of Part A(1) as a syrup. Recrystallize from hexanes to obtain white needles, m.p. 52—54°C.

2. 2-Carboamide-Thiazole

Treat 5.0 g of the product of Part A(1) with 100 ml of concentrated ammonium hydroxide solution overnight. Purge with $N_2$ for 2 hours and remove the solvent to obtain a crude product. Recrystallize using ethylacetate/pet ether to obtain the title compound, m.p. 122—124°C.

3. 4-Cyano-Thiazole

Dissolve the product of Part A(2) (2.0 g, 14.50 mmoles) in 20 ml of dry $CH_2Cl_2$ and cool in an ice bath. Add trifluoroacetic anhydride (3.65 g, 16.50 mmoles) and stir at room temperature for 3 hours. Evaporate the solvent and recrystallize from ethylacetate/pet ether to obtain the title compound, m.p. 54—56°C.

4. Ethyl-4-Cyanothiazole-2-Carboxylate

Cool diisopropyl amine (4.04 g, 40.0 mmoles) in dry THF in an ice bath under nitrogen. Add n-butyl lithium (40 ml, 40 mmoles) and stir at 0°C for 15 min. Cool the resultant reaction mixture to −78°C and add a solution of the product of part A(3) (4.0 g, 36.36 mmoles) in 20 ml THF. Stir the reaction mixture at −78°C for 30 minutes, add ethylchloroformate (3.93 g, 36.36 mmoles) and slowly warm the reaction mixture to room temperature. Add aqueous ammonium chloride and evaporate the solvent. Add $CH_2Cl_2$ (300 ml) to

the resultant residue, wash with water, dry over $MgSO_4$ and evaporate the solvent to obtain the title compound. Purify the product by column chromatography using ethylacetate/pet ether.

5. Ethyl-4-Aminomethylthiazole-2-Carboxylate

Cool a disiamylborane (17.50 mmoles) solution in an ice bath under nitrogen. Add the product of Part A(4) (1.5 g, 8.24 mmoles) in 10 ml of dry THF and let stand at 0°C for 2 days. Pour the reaction mixture into ice cold IN HCl and extract with diethyl ether. Separate the aqueous layer, treat with sodium bicarbonate, extract with 2 × 100 ml ethyl acetate, dry over $MgSO_4$ and evaporate the solvent to obtain the title compound. Use without further purification in Part B.

B. *1-N-[2-[4-[6-chloro-3,4-dihydro-3-(2-phenylethyl)-2H-1,2,4-benzothiadiazin-7-yl]sulfonyl]aminomethyl]-2-carboethoxythiazole, S,S-dioxide*

Combine the product of Preparation 2 (1.2 mmoles) and the product of Part A (1.0 mmoles) in dry DMF (1.0 ml) and cool in an ice bath. Add dropwise triethyl amine (1.5 mmoles). Stir the resulting reaction mixture at room temperature for 16 hours. Evaporate the solvent under high vacuum, take up the residue in ethylacetate (100 ml), wash with water, dry over $MgSO_4$ and evaporate the solvent. Purify the resultant residue by column chromatography.

C. *1-[N-[2-[4-[6-chloro-3,4-dihydro-3-(2-phenylethyl)-2H-1,2,4-benzothiadiazin-7-yl]sulfonyl]aminomethyl]-2-hydroxymethylthiazole, S,S-dioxide*

Dissolve the product of Part B (1.0 mmole) in 25 ml dry THF and cool to −40°C under nitrogen. Add $LiALH_4$ (1.5 mmole) and stir the resultant mixture at −40°C for 1 hour. Add $Na_2SO_4$:$10H_2O$, filter, dry over $MgSO_4$ and evaporate the solvent to obtain the title compound.

D. *1-[N-[2-[4-[6-chloro-3,4-dihydro-3-(2-phenylethyl)-2H-1,2,4-benzothiadiazin-7-yl]sulfonyl]aminomethyl]-2-bromomethylthiazole, S,S-dioxide*

Combine carbon tetrabromide (1.1 mmoles) and triphenyl phosphine (1.1 mmoles) in dry DMF (5 ml) at 0°C under nitrogen and stir at 0°C for 1/2 hour. Add a solution of the product of Part C (1.0 mmoles) in dry DMF (3 ml) and stir the reaction mixture at 0°C for 1—2 hours. (Check progress of reaction by TLC). Pour the resultant mixture into ice cold water, extract with diethyl ether (2 × 150 ml), dry over $MgSO_4$ and evaporate the solvent. Purify the resultant residue by column chromatography.

E. *Bis[[[1-(S)-(methoxycarbonyl)-ethyl]-(S)-alanyl]-cis,syn-octahydroindole-2(S)-tert-butoxycarbonyl]disulphide*

1. Cool a solution of L-cysteine (7.20 g, 0.027 moles) in 50 ml dry $CH_2Cl_2$ in an ice bath under $N_2$. Add N-methylmorpholine (16.8 g, 0.16 moles), then add a freshly prepared solution of triflate reagent (0.161 moles) (Preparation 1) in 200 ml $CH_2Cl_2$ dropwise over 2 hours. Stir the resulting reaction mixture overnight at room temperature. Wash the reaction mixture with 2 × 100 ml of water, then 2 × 100 ml of brine, dry over $MgSO_4$, and evaporate the solvent. Column chromatograph the resultant residue using 40% ethyl acetate:60% pet ether to obtain a syrup.

2. Cool a solution of the product of Part E(1) in 50 ml dioxane in an ice bath. Add 150 ml of a solution of saturated dioxane:HCl and stir at room temperature for 3 hours. Reduce the volume of the reaction mixture to 50 ml and add 150 ml of ethyl acetate to precipitate the product as the hydrochloride salt.

3. Cool a solution of the product of Part E(2) (1.0 mmole) in 10 ml dry DMF in an ice bath. Add 1-hydroxybenzotriazole hydrate (2.2 mmoles), 1-(dimethylaminopropyl)-3-ethyl-carbodiimide (3.0 mmole) and 2-carboxy-perhydroindole t-butyl ester (2.3 mmole) (Preparation 3) and stir the resulting solution at 0°C. Add triethylamine dropwise (6.0 mmoles) and stir the resulting reaction mixture over-night at room temperature. Evaporate the solvent, dissolve the residue in 200 ml of ethylacetate, wash with water, brine, and dry over $MgSO_4$. Evaporate the solvent and column chromatograph the resultant residue using 60% ethylacetate:40% pet ether.

F. Cool 5 ml of dry methanol in an ice bath under nitrogen, add sodium borohydride (10.0 mmole) and stir at 0°C for 10 minutes. Add the product of Part E (0.60 mmoles) in 5 ml dry methanol and stir the resulting mixture for 5 minutes at 0°C. Add the product of Part D (1.2 mmoles) in 5 ml THF, warm the mixture to room temperature and stir at room temperature for 2 hours. Evaporate the solvent and take up the resultant residue in ethylacetate. Extract with 10% citric acid, $NaHCO_3$ solution and brine, dry over $MgSO_4$ and evaporate the solvent. Chromatograph the residue to obtain the t-butyl ester of the title compound of Example 12.

G. Treat the product of Part F in a manner similar to that described in Example 3, Part G, to obtain the title compound. Mass spectrum m/e = 925 ($M^+$).

In a similar manner, using appropriate starting materials and reagents, the following compounds may be prepared:

1-[2-(S)-[[1-(S)-carboxy-2-[[4-[[[[6-chloro-3-(cyclopentylmethyl)-3,4-dihydro-2*H*-1,2,4-benzothiadiazin-7-yl]sulfonyl]amino]methyl]phenyl]methoxy]ethyl]amino]-1-oxopropyl]-[2S-(2α, 3aα, 7aα)]-octahydro-1*H*-indole-2-carboxylic acid, *S,S*-dioxide

1-[2-(S)-[[1-(S)-carboxy-2-[[4-[[[[6-chloro-3-(dichloromethyl)-3,4-dihydro-2*H*-1,2,4-benzothiadiazin-7-yl]sulfonyl]amino]methyl]phenyl]methoxy]ethyl]amino]-1-oxopropyl]-[2S-(2α, 3aα, 7aα)]-octahydro-1*H*-indole-2-carboxylic acid, *S,S*-dioxide

1-[2-(S)-[[1-(S)-carboxy-3-[4-[[[[6-chloro-3,4-dihydro-3-(2-phenylethyl)-2*H*-1,2,4-benzothiadiazin-7-yl]-sulfonyl]amino]methyl]phenyl]propyl]amino]-1-oxopropyl]-[2S-(2α, 3aα, 7aα)]-octahydro-1*H*-indole-2-carboxylic acid, *S,S*-dioxide

1-[2-(S)-[[1-(S)-carboxy-3-[4-[[[[6-chloro-3-(cyclopentylmethyl)-3,4-dihydro-2H-1,2,4-benzothiadiazin-7-yl]sulfonyl]amino]methyl]phenyl]propyl]amino]-1-oxopropyl]-[2S-(2α, 3aα, 7aα)]-octahydro-1*H*-indole-2-carboxylic acid, *S,S*-dioxide

1-[2-(S)-[[1-(S)-carboxy-3-[4-[[[[6-chloro-3-(dichloromethyl)-3,4-dihydro-2*H*-1,2,4-benzothiadiazin-7-yl]-sulfonyl]amino]methyl]phenyl]propyl]amino]-1-oxopropyl]-[2S-(2α, 3aα, 7aα)]-octahydro-1*H*-indole-2-carboxylic acid, *S,S*-dioxide

1-[2-(S)-[[1-(S)-carboxy-2-[4-[[[[6-chloro-3,4-dihydro-3-(2-phenylethyl)-2*H*-1,2,4-benzothiadiazin-7-yl]sulfonyl]amino]methyl]phenyl]ethyl]amino]-1-oxopropyl]-[2S-(2α, 3aα, 7aα)]-octahydro-1*H*-indole-2-carboxylic acid, *S,S*-dioxide

1-[N-[1-(S)-carboxy-3-[4-[[[[6-chloro-3,4-dihydro-3-(2-phenylethyl)-2*H*-1,2,4-benzothiadiazin-7-yl]sulfonyl]amino]methyl]phenyl]propyl]-(L)-alanyl]-(L)-proline, *S,S*-dioxide

1-[N-[1-(S)-carboxy-3-[4-[[[[6-chloro-3-(cyclopentylmethyl)-3,4-dihydro-2*H*-1,2,4-benzothiadiazin-7-yl]sulfonyl]amino]methyl]phenyl]propyl]-(L)-alanyl]-(L)-proline, *S,S*-dioxide

1-[N-[1-(S)-carboxy-3-[4-[[[[6-chloro-3-(dichloromethyl)-3,4-dihydro-2*H*-1,2,4-benzothiadiazin-7-yl]-sulfonyl]amino]methyl]phenyl]propyl]-(L)-alanyl]-(L)-proline, *S,S*-dioxide

1-[N-[1-(S)-carboxy-2-[4-[[[[6-chloro-3,4-dihydro-3-(2-phenylethyl)-2*H*-1,2,4-benzothiadiazin-7-yl]-sulfonyl]amino]methyl]phenyl]ethyl]-(L)-alanyl]-(L)-proline, *S,S*-dioxide

1-[N-[1-(S)-carboxy-3-[4-[[[[6-chloro-3,4-dihydro-3-(2-phenylethyl)-2*H*-1,2,4-benzothiadiazin-7-yl]sulfonyl]amino]methyl]phenyl]propyl]-(L)-alanyl]-(L)-(4-cyclohexyl)proline, *S,S*-dioxide

7-[2-(S)-[[1-(S)-carboxy-3-[4-[[[[6-chloro-3,4-dihydro-3-(2-phenylethyl)-2*H*-1,2,4-benzothiadiazin-7-yl]-sulfonyl]amino]methyl]phenyl]propyl]amino]-1-oxopropyl]-1,4-dithia-7-azaspiro[4.4]nonane-8-(S)-carboxylic acid, *S,S*-dioxide

1-[2-(S)-[[1-(S)-carboxy-3-[4-[[[[6-chloro-3,4-dihydro-3-(2-phenylethyl)-2*H*-1,2,4-benzothiadiazin-7-yl]sulfonyl]amino]methyl]phenyl]propyl]amino]-1-oxopropyl]-1*H*-2,3-dihydroindole-2-(S)-carboxylic acid *S,S*-dioxide

1-[2-(S)-[[2-[[4-[[[[6-chloro-3,4-dihydro-3-(2-phenylethyl)-2*H*-1,2,4-benzothiadiazin-7-yl]sulfonyl]-amino]methyl]phenyl]methoxy]-1-(S)-[[(2,2-dimethyl-1,3-dioxolan-4-yl)methoxy]carbonyl]ethyl]amino] 1-oxopropyl]-[2S-(2α, 3aα, 7aα)]-octahydro-1*H*-indole-2-carboxylic acid, *S,S*-dioxide

1-[2-(S)-[[3-[4-[[[[6-chloro-3,4-dihydro-3-(2-phenylethyl)-2*H*-1,2,4-benzothiadiazin-7-yl]sulfonyl]amino]-methyl]phenyl]-1-(S)-[[(2,2-dimethyl-1,3-dioxolan-4-yl)methoxy]carbonyl]propyl]amino]-1-oxopropyl]-[2S-(2α, 3aα, 7aα)]-octahydro-1*H*-indole-2-carboxylic acid, *S,S*-dioxide

1-[2-(S)-[[3-[4-[[[[6-chloro-3,4-dihydro-3-(2-phenylethyl)-2*H*-1,2,4-benzothiadiazin-7-yl]sulfonyl]amino]-methyl]phenyl]-1-(S)-(ethoxycarbonyl)propyl]amino]-1-oxopropyl]-[2S-(2α, 3aα, 7aα)]-octahydro-1*H*-indole-2-carboxylic acid, *S,S*-dioxide

1-[N-[3-[4-[[[[6-chloro-3,4-dihydro-3-(2-phenylethyl)-2*H*-1,2,4-benzothiadiazin-7-yl]sulfonyl]amino]-methyl]phenyl]-1-(S)-(ethoxycarbonyl)propyl]-(L)-alanyl]-(L)-proline, *S,S*-dioxide

1-[N-[3-[4-[[[[6-chloro-3,4-dihydro-3-(2-phenylethyl)-2*H*-1,2,4-benzothiadiazin-7-yl]sulfonyl]amino]-methyl]phenyl]-1-(S)-[(2-phenoxyethoxy)carbonyl]propyl]-(L)-alanyl]-(L)-proline, *S,S*-dioxide.

1-[N-[3-[4-[[[[6-chloro-3,4-dihydro-3-(2-phenylethyl)-2*H*-1,2,4-benzothiadiazin-7-yl]sulfonyl]amino]-methyl]phenyl]-1-(S)-[(2,3-dihydroxypropoxy)carbonyl]propyl]-(L)-alanyl]-(L)-proline, *S,S*-dioxide.

1-[N-[3-[4-[[[[6-chloro-3-cyclopentylmethyl)-3,4-dihydro-2*H*-1,2,4-benzothiadiazin-7-yl]sulfonyl]amino]-methyl]phenyl]-1-(S)-(ethoxycarbonyl)propyl]-(L)-alanyl]-(L)-proline, *S,S*-dioxide

1-[N-[3-[4-[[[[6-chloro-3,4-dihydro-3-(2-phenylethyl)-2*H*-1,2,4-benzothiadiazin-7-yl]sulfonyl]amino]-methyl]phenyl]-1-(S)-[[(2,2-dimethyl-1,3-dioxolan-4-yl)methoxy]carbonyl]propyl]-(L)-alanyl]-(L)-proline, *S,S*-dioxide

1-[N-[3-[4-[[[[6-chloro-3-cyclopentylmethyl)-3,4-dihydro-2*H*-1,2,4-benzothiadiazin-7-yl]sulfonyl]amino]-methyl]phenyl]-1-(S)-[[(2,2-dimethyl-1,3-dioxolan-4-yl)methoxy]carbonyl]propyl]-(L)-alanyl]-(L)-proline, *S,S*-dioxide

1-[N-[3-[4-[[[[6-chloro-3-cyclopentylmethyl)-3,4-dihydro-2*H*-1,2,4-benzothiadiazin-7-yl]sulfonyl]amino]-methyl]phenyl]-1-(S)-[(2-phenoxyethoxy)carbonyl]propyl]-(L)-alanyl]-(L)-proline, *S,S*-dioxide

1-[N-[3-[4-[[[[6-chloro-3-cyclopentylmethyl)-3,4-dihydro-2*H*-1,2,4-benzothiadiazin-7-yl]sulfonyl]amino]-methyl]phenyl]-1-(S)-[(2,3-dihydroxypropoxy)carbonyl]propyl]-(L)-alanyl]-(L)-proline, *S,S*-dioxide

1-[2-[[2-[[4-[[[[4-chloro-3-[[(phenylmethyl)amino]sulfonyl]phenyl]sulfonyl]amino]methyl]phenyl]-methoxy]-1-(S)-(ethoxycarbonyl)ethyl]amino]-1-oxopropyl]-[2S-(2α,3aα,7aα)]-octahydro-1*H*-indole-2-carboxylic acid

1-[2-[-(S)-carboxy-[2-[[4-[[[[4-chloro-3-[[(phenylmethyl)amino]sulfonyl]phenyl]sulfonyl]amino]methyl]-phenyl]methoxy]ethyl]amino]-1-oxopropyl]-[2S-(2α,3aα,7aα)]-octahydro-1*H*-indole-2-carboxylic acid

1-[N-[2-[[4-[[[[4-chloro-3-[[(phenylmethyl)amino]sulfonyl]phenyl]sulfonyl]amino]methyl]phenyl]-methoxy]-1-(S)-(ethoxycarbonyl)ethyl]-(L)-alanyl]-(L)-proline

1-[N-[-(S)-carboxy-2-[[4-[[[[4-chloro-3-[[(phenylmethyl)amino]sulfonyl]phenyl]sulfonyl]amino]methyl]-phenyl]methoxy]ethyl]-(L)-alanyl]-(L)-proline

1-[2-[[2-[[4-[[[[3-(aminosulfonyl)-4-chlorophenyl]sulfonyl]amino]methyl]phenyl]methoxy]-1-(S)-(ethoxycarbonyl)ethyl]amino]-1-oxopropyl]-[2S-(2α,3aα,7aα)]-octahydro-1*H*-indole-2-carboxylic acid

1-[2-[[2-[[4-[[[[3-(aminosulfonyl)-4-chlorophenyl]sulfonyl]amino]methyl]phenyl]methoxy]-1-(S)-carboxyethyl]amino]-1-oxopropyl]-(2S-(2α,3aα,7aα)]-octahydro-1*H*-indole-2-carboxylic acid

1-[N-[2-[[4-[[[[3-(aminosulfonyl)-4-chlorophenyl]sulfonyl]amino]methyl]phenyl]methoxy]-1-(S)-(ethoxycarbonyl)ethyl]-(L)-alanyl-(L)-proline

1-[N-[2-[[4-[[[[3-(aminosulfonyl)-4-chlorophenyl]sulfonyl]amino]methyl]phenyl]methoxy]-1-(S)-(ethoxycarbonyl)ethyl]-(L)-lysyl]-(L)-proline

1-[N-[2-[[4-[[[[3-(aminosulfonyl)-4-chlorophenyl]sulfonyl]amino]methyl]phenyl]methoxy]-1-(S)-carboxyethyl]-(L)-alanyl]-(L)-proline

1-[N-[2-[[4-[[[[3-(aminosulfonyl)-4-chlorophenyl]sulfonyl]amino]methyl]phenyl]methoxy]-1-(S)-carboxyethyl]-(L)-lysyl]-(L)-proline

1-[N-[2-[[4-[[[[3-(aminosulfonyl)-4-chlorophenyl]sulfonyl]amino]methyl]phenyl]methoxy]-1-(S)-[[(2,2-dimethyl-1-oxopropoxy)methoxy]carbonyl]ethyl]-(L)-alanyl]-(L)-proline

1-[N-[3-[4-[[[[6-chloro-3,4-dihydro-2*H*-1,2,4-benzothiadiazin-7-yl]sulfonyl]amino]methyl]phenyl]-1-(S)-(ethoxycarbonyl)propyl]-(L)-alanyl]-(L)-proline, *S,S*-dioxide

1-[N-[3-[4-[[[[6-chloro-3,4-dihydro-2*H*-1,2,4-benzothiadiazin-7-yl]sulfonyl]amino]methyl]phenyl]-1-(S)-[[2,2-dimethyl-1-oxopropoxy)methoxy]carbonyl]propyl]-(L)-alanyl]-(L)-proline, *S,S*-dioxide

1-[N-[3-[4-[[[[6-chloro-3,4-dihydro-2*H*-1,2,4-benzothiadiazin-7-yl]sulfonyl]amino]methyl]phenyl]-1-(S)-(ethoxycarbonyl)propyl]-(L)-lysyl]-(L)-proline, *S,S*-dioxide

1-[N-[1-(S)-carboxy-3-[4-[[[[6-chloro-3,4-dihydro-2*H*-1,2,4-benzothiadiazin-7-yl]sulfonyl]amino]-methyl]phenyl]propyl]-(L)-alanyl]-(L)-proline, *S,S*-dioxide

1-[N-[1-(S)-carboxy-3-[4-[[[[6-chloro-3,4-dihydro-2*H*-1,2,4-benzothiadiazin-7-yl]sulfonyl]amino]-methyl]phenyl]propyl]-(L)-lysyl]-(L)-proline, *S,S*-dioxide

1-[N-[1-(S)-carboxy-3-[4-[[[[6-chloro-3-(chloromethyl)-3,4-dihydro-2*H*-1,2,4-benzothiadiazin-7-yl]sulfonyl]amino]methyl]phenyl]propyl]-(L)-alanyl-(L)-proline, *S,S*-dioxide

1-[N-[1-(S)-carboxy-3-[4-[[[[6-chloro-3-(chloromethyl)-3,4-dihydro-2*H*-1,2,4-benzothiadiazin-7-yl]sulfonyl]amino]methyl]phenyl]propyl]-(L)-lysyl]-(L)-proline, *S,S*-dioxide

1-[N-[3-[4-[[[[6-chloro-3-(chloromethyl)-3,4-dihydro-2*H*-1,2,4-benzothiadiazin-7-yl]sulfonyl]amino]-methyl]phenyl]-1-(S)-[[(2,2-dimethyl-1-oxopropoxy)methoxy]carbonyl]propyl]-(L)-alanyl]-(L)-proline, *S,S*-dioxide

1-[N-[3-[4-[[[[6-chloro-3-(chloromethyl)-3,4-dihydro-2*H*-1,2,4-benzothiadiazin-7-yl]sulfonylamino]-methyl]phenyl]-1-(S)-(ethoxycarbonyl)propyl]-(L)-alanyl]-(L)-proline, *S,S*-dioxide

1-[2-(S)-[[2-[[4-[[[[6-chloro-3-(chloromethyl)-3,4-dihydro-2*H*-1,2,4-benzothiadiazin-7-yl]sulfonyl]-amino]methyl]phenyl]methoxy]-1-(S)-(ethoxycarbonyl)ethyl]amino]-1-oxopropyl]-[2S-(2α,3aα,7aα)]-octahydro-1*H*-indole-2-carboxylic acid, *S,S*-dioxide

1-[2-(S)-[[1-(S)-carboxy-2-[4-[[[[6-chloro-3-chloromethyl-3,4-dihydro-2*H*-1,2,4-benzothiadiazin-7-yl]-sulfonyl]amino]methyl]phenyl]ethyl]amino]-1-oxopropyl]-[2S-(2α,3aα,7aα)]-octahydro-1*H*-indole-2-carboxylic acid, *S,S*-dioxide

1-[2-(S)-[[1-(S)-carboxy-2-[[4-[[[[6-chloro-3-chloromethyl-3,4-dihydro-2*H*-1,2,4-benzothiadiazin-7-yl]-sulfonyl]amino]methyl]phenyl]methoxy]ethyl]amino]-1-oxopropyl]-[2S-(2α,3aα,7aα)]-octahydro-1*H*-indole-2-carboxylic acid, *S,S*-dioxide

1-[2-(S)-[[1-(S)-carboxy-2-[[4-[[[[6-chloro-3,4-dihydro-2*H*-1,2,4-benzothiadiazin-7-yl]sulfonyl]amino]-methyl]phenyl]methoxy]ethyl]amino]-1-oxopropyl]-[2S-(2α,3aα,7aα)]-octahydro-1*H*-indole-2-carboxylic acid, *S,S*-dioxide

1-[N-[1-(S)-carboxy-5-[[6-chloro-3,4-dihydro-2*H*-1,2,4-benzothiadiazin-7-yl]sulfonyl]amino-3-pentyn-yl]-(L)-alanyl]-(L)-proline, *S,S*-dioxide

1-[N-[5-[[6-chloro-3,4-dihydro-2*H*-1,2,4-benzothiadiazin-7-yl]-sulfonyl]amino]-1-(S)-(ethoxycarbonyl-3-pentyn-yl]-(L)-alanyl]-(L)-proline, *S,S*-dioxide

1-[4-carboxy-5-[[4-[[[[6-chloro-3-(cyclopentylmethyl)-3,4-dihydro-2*H*-1,2,4-benzothiadiazin-7-yl]-sulfonyl]amino]methyl]phenyl]methoxy]-1-oxopentyl]-octahydro-1*H*-indole-2-carboxylic acid, *S,S*-dioxide

1-[2-(S)-[1-(S)-carboxy-3-[4-[[[[6-chloro-3,4-dihydro-3-(2-phenylethyl)-2*H*-1,2,4-benzothiadiazin-7-yl]sulfonyl]amino]methyl]phenyl]propoxy]-1-oxopropyl]-(L)-proline, *S,S*-dioxide

1-N-[N-[3-[5-[[[[6-chloro-3,4-dihydro-3-(2-phenylethyl)-2*H*-1,2,4-benzothiadiazin-7-yl]sulfonyl]amino]-methyl]-1*H*-imidazol-2-yl]-1-(ethoxycarbonyl)propyl]-(L)-alanyl]-(L)-proline, *S,S*-dioxide

1-N-[N-[3-[5-[[[[6-chloro-3,4-dihydro-3-(2-phenylethyl)-2*H*-1,2,4-benzothiadiazin-7-yl]sulfonyl]amino]-methyl]-2-thiazolyl]-1-(ethoxycarbonyl)propyl]-(L)-alanyl]-(L)-proline, *S,S*-dioxide

1-[[[N-[2-[4-[[[[3-butyl-6-chloro-3,4-dihydro-2*H*-1,2,4-benzothiadiazin-7-yl]sulfonyl]amino]methyl]-2-thiazol yl]methyl]thio]-1-(S)-(methoxycarebonyl)ethyl]-(L)-alanyl-(L)-proline, *S,S*-dioxide

1-[N-[1-(S)-carboxy-4-[4-[[[[6-chloro-3,4-dihydro-3-(2-phenylethyl)-2*H*-1,2,4-benzothiadiazin-7-yl]-sulfonyl]amino]methyl]phenyl]butyl]-(L)lysyl]-(L)-proline, *S,S*-dioxide

7-[2-(S)-[[4-[4-[[[[3-butyl-6-chloro-3,4-dihydro-2*H*-1,2,4-benzothiadiazin-7-yl]sulfonylamino]methyl]-phenyl]-1-(S)-carboxybutyl]amino]-1-oxopropyl]-1,4-dithia-7-azaspiro[4.4]-nonane-8-(S)-carboxylic acid, *S,S*-dioxide

N-[4-[4-[[[[6-chloro-3,4-dihydro-3-(2-phenylethyl)-2*H*-1,2,4-benzothiadiazin-7-yl]sulfonyl]amino]-methyl]phenyl]-1-(S)-(ethoxycarbonyl)butyl]-(L)-lysyl-(L)-proline, *S,S*-dioxide

1-[[1-(S)-carboxy-4-[4-[[[[6-chloro-3,4-dihydro-3-(2-phenylethyl)-2*H*-1,2,4-benzothiadiazin-7-yl]-sulfonyl]amino]methyl]phenyl]butyl]-L-alanyl]-(L)-proline, *S,S*-dioxide

1-2-(S)-[[3-[4-[[[[6-chloro-3,4-dihydro-3-[2-(2-pyridinyl)ethyl]-2*H*-1,2,4-benzothiadiazin-7-yl]sulfonyl]-amino]methyl]-phenyl]-1-(S)-(ethoxycarbonyl)propyl]amino]-1-oxopropyl]-[2S-(2α,3aα,7aα)]-octahydro-1*H*-indole-2-carboxylic acid, *S,S*-dioxide

1-[2-(S)-[[1-(S)-carboxy-4-[[[[3-butyl-6-chloro-3,4-dihydro-2*H*-1,2,4-benzothiadiazin-7-yl]sulfonyl]-amino]methyl]phenyl]butyl]amino]-1-oxopropyl]-[2S-(2α,3aα,7aα)]-octahydro-1*H*-indole-2-carboxylic acid, *S,S*-dioxide

1-[N-[3-[4-[[[[3-butyl-6-chloro-3,4-dihydro-2*H*-1,2,4-benzothiadiazin-7-yl]sulfonyl]amino]methyl]-phenyl]-1-(S)-(ethoxycarbonyl)propyl]-(L)-alanyl]-(L)-proline, *S,S*-dioxide

1-[N-[2-(S)-[[4-[4-[[[[3-butyl-6-chloro-3,4-dihydro-2*H*-1,2,4-benzothiadiazin-7-yl]sulfonyl]amino]-methyl]phenyl]-1-(S)-(ethoxycarbonyl)butyl]-(L)-alanyl]-(L)-proline, *S,S*-dioxide

1-[2-(S)-[[4-[4-[[[[6-chloro-3,4-dihydro-3-butyl-2*H*-1,2,4-benzothiadiazin-7-yl]sulfonyl]amino]methyl]-phenyl]-1-(S)-(ethoxycarbonyl)butyl]amino]-1-oxopropyl]-[2S-(2α,3aα,7aα)]-octahydro-1*H*-indole-2-carboxylic acid, *S,S*-dioxide

1-[N-[2-[[4-[[[[3-butyl-6-chloro-2,3-dihydro-2*H*-1,2,4-benzothiadiazin-7-yl]sulfonyl]amino]methyl]-phenyl]methoxy]-1-(S)-(ethoxycarbonyl)ethyl]-(L)-alanyl]-(L)-proline, *S,S*-dioxide

1-[N-[2-[[[4-[[[[6-chloro-3,4-dihydro-3-(2-phenylethyl)-2*H*-1,2,4-benzothiadiazin-7-yl]sulfonyl]amino]-methyl]phenyl]methyl]thio]-1-(S)-(ethoxycarbonyl)ethyl]-(L)-alanyl]-(L)-proline, S,S-dioxide

1-[N-[2-[[[4-[[[[6-chloro-3,4-dihydro-3-butyl-2*H*-1,2,4-benzothiadiazin-7-yl]sulfonyl]amino]methyl]-phenyl]methyl]thio]-1-(S)-(methoxycarbonyl)ethyl]-(L)-alanyl]-(L)-proline, *S,S*-dioxide

1-[N-[2-[[[4-[[[[6-chloro-3,4-dihydro-3-(chloromethyl)-2*H*-1,2,4-benzothiadiazin-7-yl]sulfonyl]amino]-methyl]phenyl]methyl]thio]-1-(S)-(ethoxycarbonyl)ethyl]-(L)-alanyl]-(L)-proline, *S,S*-dioxide

1-[N-[2-[[[4-[[[[6-chloro-3,4-dihydro-3-(cyclopentylmethyl)-2*H*-1,2,4-benzothiadiazin-7-yl]sulfonyl]-amino]methyl]phenyl]methyl]thio]-1-(S)-(ethoxycarbonyl)ethyl]-(L)-alanyl]-(L)-proline, *S,S*-dioxide

1-[2-[[[2-[[4-[[[[3-butyl-6-chloro-3,4-dihydro-2*H*-1,2,4-benzothiadiazin-7-yl]sulfonyl]amino]methyl]-phenyl]methyl]thio]-1-(ethoxycarbonyl)ethyl]amino]-1-oxopropyl]-[2S-(2α,3aα,7aα)]-octahydro-1*H*-indole-2-carboxylic acid, *S,S*-dioxide

1-[N-[3-[5-[[[[6-chloro-3,4-dihydro-3-(2-phenylethyl)-2*H*-1,2,4-benzothiadiazin-7-yl]sulfonyl]amino]-methyl]-2-pyridinyl]-1-(ethoxycarbonyl)propyl]-(L)-alanyl]-(L)-proline, *S,S*-dioxide

1-[N-[3-[2-[[[[6-chloro-3,4-dihydro-3-(2-phenylethyl)-2*H*-1,2,4-benzothiadiazin-7-yl]sulfonyl]amino]-methyl]-5-pyridinyl]-1-(ethoxycarbonyl)propyl]-(L)-alanyl]-(L)-proline, *S,S*-dioxide

1-[N-[N-[3-[4-[[[[6-chloro-3,4-dihydro-3-(2-phenylethyl)-2*H*-1,2,4-benzothiadiazin-7-yl]sulfonyl]amino]-methyl]-2-thiazolyl]-1-(ethoxycarbonyl)propyl]-(L)-alanyl]-(L)-proline, *S,S*-dioxide

1-[N-[N-[3-[4-[[[[6-chloro-3,4-dihydro-3-(2-phenylethyl)-2*H*-1,2,4-benzothiadiazin-7-yl]sulfonyl]amino]-methyl]-2-oxazolyl]-1-(ethoxycarbonyl)propyl]-(L)-alanyl]-(L)-proline, *S,S*-dioxide

1-[N-[N-[4-[4-[[[[6-chloro-3,4-dihydro-3-[2-(2-pyridinyl)ethyl]-2*H*-1,2,4-benzothiadiazin-7-yl]sulfonyl]-amino]methyl]phenyl]-1-(S)-(ethoxycarbonyl)butyl]-(L)-alanyl]-(L)-proline, *S,S*-dioxide

1-[N-[N-[3-[5-[[[[6-chloro-3,4-dihydro-3-(2-phenylethyl)-2*H*-1,2,4-benzothiadiazin-7-yl]sulfonyl]amino]-methyl]-1*H*-imidazol-2-yl]-1-(ethoxycarbonyl)propyl]-(L)-alanyl]-(L)]-proline, *S,S*-dioxide

1-[N-[N-[3-[5-[[[[6-chloro-3,4-dihydro-3-(2-phenylethyl)-2*H*-1,2,4-benzothiadiazin-7-yl]sulfonyl]amino]-methyl]-2-thiazolyl]-1-(ethoxycarbonyl)propyl]-(L)-alanyl]-(L)-proline, *S,S*-dioxide

1-[N-[3-[4-[[[[6-chloro-3,4-dihydro-3-(2-phenylethyl)-2*H*-1,2,4-benzothiadiazin-7-yl]sulfonyl]amino]-methyl]phenyl]-1-(*S*)-(ethoxycarbonyl)butyl]-L-alanyl-(L)-proline S,S-dioxide

1-[2-(*S*)-[[1-(*S*)-carboxy-3-[4-[[[[6-chloro-3,4-dihydro-3-(2-phenylethyl)-2*H*-1,2,4-benzothiadiazin-7-yl]-sulfonyl]amino]methyl]phenyl]butyl]amino]-1-oxopropyl]-[2-(*S*)-(2α,3aα,7aα)]-octahydro-1*H*-indole-2-carboxylic acid, S,S-dioxide

1-[2-(*S*)-[[3-[4-[[[[6-chloro-3,4-dihydro-3-(2-phenylethyl)-2*H*-1,2,4-benzothiadiazin-7-yl]sulfonyl]amino]-methyl]phenyl]-1-(*S*-(ethoxycarbonyl)butyll]amino]-1-oxopropyl]-[2*S*-(2α,3aα,7aα)]-octahydro-1*H*-indole-2-carboxylic acid, S,S-dioxide

1-[N-[1-(*S*-carboxy-3-[4-[[[[6-chloro-3,4-dihydro-3-(2-phenylethyl)-2*H*-1,2,4-benzothiadiazin-7-yl]-sulfonyl]amino]methyl]phenyl]propyl]-L-alanyl]-(L)-proline, S,S-dioxide

1-[N-[3-[4-[[[-6-chloro-3,4-dihydro-3-(2-phenylethyl)-2*H*-1,2,4-benzothiazin-7-yl]sulfonyl]amino]-methyl]phenyl]-1-(*S*)-(ethoxycarbonyl)propyl]-L-alanyl]-(L)-proline, S,S,-dioxide

1-[2-(*S*)-[[1-(*S*)-carboxy-3-[4-[[[6-chloro-3,4-dihydro-3-(chloromethyl)-2*H*-1,2,4-benzothiadiazin-7-yl]sulfonyl]amino]methyl]phenyl]propyl]amino]-1-oxopropyl]-[2-(*S*)-(2α,3aα,7aα)]-octahydro-1*H*-indole-2-carboxylic acid, S,S-dioxide

1-[2-(S)-[[3-[4-[[[6-chloro-3,4-dihydro-3-(chloromethyl)-2*H*-1,2,4-benzothiadiazin-7-yl]sulfonyl]amino]-methyl]phenyl]-1-(S)-(ethoxycarbonyl)propyl]amino]-1-oxopropyl]-(2*S*-(2α,3aα,7aα)]-octahydro-1*H*-indole-2-carboxylic acid, S,S-dioxide

1-[2-(S)-[[3-[4-[[[6-chloro-3,4-dihydro-3-[2-(2-pyridinyl)ethyl]-2*H*-1,2,4-benzothiadiazin-7-yl]-

sulfonyl]amino]methyl]phenyl]-1(*S*)-(ethoxycarbonyl)propyl]amino]-1-oxopropyl]-[2*S*-(2α,3aα,7aα)]-octahydro-1*H*-indole-2-carboxylic acid, S,S-dioxide

(1,1-dimethylethyl) 1-[2-(S)-[[2-[[[4-[[[[6-chloro-3,4-dihydro-3-(2-phenylethyl)-2*H*-1,2,4-benzothiadiazin-7-yl]sulfonyl]amino]methyl]-2-thiazolyl]methyl]thiol]-1(*S*)-(methoxycarbonyl)ethyl]-amino]-1-oxopropyl]-[2S-(2α,3aα,7aα)]-octahydro-1H-indole-2-carboxylate, S,S-dioxide

1-[2-(S)[[2-[[[4-[[[[6-chloro-3,4-dihydro-3-(2-phenylethyl)-2*H*-1,2,4-benzothiadiazin-7-yl]sulfonyl]-amino]methyl]-2-thiazolyl]methyl]thio]-1(*S*)-(methoxycarbonyl)ethyl]amino]-1-oxopropyl]-[2S-[2α,3aα,7aα)]-octahydro-1H-indole-2-carboxylic acid, S,S-dioxide

1-[2-(S)-[[1-carboxy-2-[[[4-[[[[6-chloro-3,4-dihydro-3-(2-phenylethyl)-2*H*-1,2,4-benzothiadiazin-7-yl]sulfonyl]amino]methyl]-2-thiazolyl]methyl]thio]ethyl]amino]-1-oxopropyl]-[2S-(2α,3aα,7aα)]-octahydro-1H-indole-2-carboxylic acid, S,S-dioxide

1-[2-(S)-[[1-carboxy-2-[[[5-[[[[6-chloro-3,4-dihydro-3-(2-phenylethyl)-2*H*-1,2,4-benzothiadiazin-7-yl]sulfonyl]amino]methyl]-2-thiazolyl]methyl]thio]ethyl]amino]-1-oxopropyl]-[2S-(2α,3aα,7aα)]-octahydro-1H-indole-2-carboxylic acid, S,S-dioxide

The compounds of this invention are useful in view of their pharmacological properties. In particular, representative compounds of the invention possess activity as antihypertensive agents, as evidenced by their ability to reduce blood pressure in mammals in which the blood pressure has become abnormally elevated. For example, compounds of this invention lower blood pressure in the spontaneously hypertensive rat (SHR) model.

The hypertensive activity of the compounds of the invention is illustrated by reference to the following compounds for which the activity of the compounds in the spontaneously hypertensive rat was measured using the methodology of D. J .S. Chiv, S. Vemulapalli and A. Barnett J. Pharm. Pharmacol., *37*, 105—109 (1985).

Compound A:

1-[N-[3-[4-[[[[6-chloro-3,4-dihydro-3-(2-phenylethyl)-2*H*-1,2,4,-benzothiadiazin-7-yl]sulfonyl]amino]-methyl]phenyl]-1-(*S*)-(ethoxycarbonyl)butyl]-L-alanyl]-(L)-proline-S,S-dioxide

Compound B:

1-[2-(S)-[[2-[[[4-[[[[6-chloro-3,4-dihydro-3-butyl-2*H*-1,2,4-benzothiadiazin-7-yl]sulfonyl]amino]ethyl]-phenyl]methyl]thio]-1(S)-(methoxycarbonyl)ethyl]amino]-1-oxopropyl]-[2S-(2α,3aα,7aα)-octahydro-1*H*-indole-2-carboxylic acid, S,S-dioxide

For compound A at a dose of 30 mg/kg (mpk) a reduction in mean blood pressure measured over a period of 5 hours, of 36 mbar (27 mm Hg) was obtained and for Compound B, at a dose of 10 mg/kg (mpk), a reduction of 29.3 mbar (22 mm Hg) was obtained.

Representative compounds of this invention also exhibit activity as diuretic agents. Further, they exhibit angiotensin converting enzyme inhibitory activity and are thus contemplated for use in treating cardiovascular disorders, for example congestive heart failure, in the same manner as other ACE inhibitors such as captopril and enalapril. In addition, compounds of this invention may be used in the treatment of glaucoma by topical application.

The compounds of this invention can be combined with pharmaceutical carriers and administered in a variety of well-known pharmaceutical forms suitable for oral or parenteral administration to provide compositions useful in the treatment of cardiovascular disorders and particularly mammalian hypertension.

The daily antihypertensive dose of the compounds of this invention will be typically in the range of about 1 to about 25 mg/kg, of mammalian weight, administered in single or divided doses. The exact dose to be administered is determined by the attending clinician and is dependent upon the potency of the administered compound, i.e. where the particular compound lies within the above range, as well as upon the age, weight and condition of the individual.

Generally, in treating humans having hypertension, the compounds of this invention may be administered to patients in need of such treatment in dosage range of about 5 to about 500 mg per patient generally given several times a day, thus giving a total daily dose of from about 5 to about 2000 mg per day.

The antihypertensive compositions containing the compounds of this invention will preferably contain from about 5 to about 250 mg of the active compound per dosage unit.

The compositions of the present invention are most preferably administered orally. Typical formulations for oral administration are those such as tablets, capsules, syrups, elixirs or suspensions. Typical injectable formulations include solutions and suspensions. Also contemplated are mechanical delivery systems, e.g. transdermal dosage forms.

The typical acceptable pharmaceutical carriers for use in the formulations described herein are exemplified by: sugars such as lactose, sucrose, mannitol and sorbitol; starches such as corn starch tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone, polyvinyl alcohol; stearic acid; alkaline earth metal stearates such as magnesium stearate and calcium stearate; stearic acid; vegetable oils such as peanut oil, cottonseed oil, sesame oil, olive oil and corn oil; non-ionic, cationic and anionic surfactants;

ethylene glycol polymers; betacyclodextrin; fatty alcohols and hydrolyzed cereal solids; as well as other non-toxic compatible fillers, binders, disintegrants, buffers, preservatives, antioxidants; lubricants, flavoring agents, and the like commonly used in pharmaceutical formulations.

In their opthalmological use, the compounds of this invention are typically administered as topical preparations such as solutions, suspensions, ointments and solid inserts. Formulations of these compounds may contain from 0.00001 to 1.0%, preferably 0.00001 to 0.1%, and especially 0.00001 to 0.001% of medicament. Other concentrations may be employed provided the dose is effective in lowering intraocular pressure. As a unit dosage form, between 0.005 mcg to 0.5 mg, preferably 0.005 mcg to 50 mcg, and especially 0.005 mcg to 0.5 mcg of the active compound is applied to the human eye, generally on a daily basis. Individual dosage requirements are variable; however, and must be administered by the attending clinician on the basis of the severity of the disease and the condition and response of the patient.

To prepare suitable dosage forms, the active compounds may be conveniently admixed with a non-toxic pharmaceutically acceptable carrier suitable for topical ophthalmolgic administration.

When topically administered to the eye, the compounds of the invention reduce intraocular pressure (IOP). For example, 1-[2-(S)-carboxy-5-[[6-chloro-3-chloromethyl-3,4-dihydro-2*H*-1,2,4-benzothiadiazinyl-7-yl]sulfonylamino]-pentyl]amino]-1-oxopropyl]-2S-[2α,3aα,7aα)]-octahydro-1*H*-indole-2-carboxylic acid, *S,S*-dioxide caused falls in IOP of a magnitude similar to those produced by the anti-glaucoma agent timolol when each were administered at concentrations of 0.001 and 0.5 (w/v%), respectively, and tested by the following procedure:

Male New Zealand white rabbits having a normal IOP are conditioned to the laboratory setting for at least one 4 hour period before being used to study drug effects. A Makay-Marq applanation tonometer is used to measure IOP. Readings, in mbar (mm Hg), are taken in triplicate and the average is recorded.

Rabbits are restrained in a cloth sack 2 minutes prior to IOP determination. The left lower eyelid is retracted to form a pouch and 1 drop of a local anesthetic is irrigated over the cornea. The lower eyelid is then held closed over the eye for about 1 minute. Corneal anesthesia is repeated before each set of IOP determinations. Readings are taken just before dosing with drug (0 time) and at hourly intervals thereafter. Drugs are administered in 50 µl volume in the same manner as the anesthetic.

As to the toxicity of the compounds of the invention, they are non-toxic at the therapeutic dose.

In the following examples the "active ingredient" is 1 - [2(S) - [[1(S) - carboxy - 2 - [4 - [[[6 - chloro - 3,4 - dihydro - 3 - (2 - phenylethyl) - 2*H* - 1,2,4 - benzothiadiazin - 7 - yl)sulfonylamino]methyl]phenyl-methoxy]ethyl]amino] - 1 - oxopropyl] - [2(S) - (2α,3aα,7aα)] - octahydro - 1*H* - indole - 2 - carboxylic acid, *S,S*-dioxide. It is contemplated, however, that this compound may be replaced by equally effective quantities of other compounds within the scope of formula I.

Example 13

| Capsule | Amount (mg) | |
|---|---|---|
| Active ingredient | 250.0 | 125.0 |
| Lactose | 173.0 | 86.5 |
| Corn Starch | 75.0 | 37.5 |
| Magnesium Stearate | 2.0 | 1.0 |
| | 500.00 | 500.00 |

Blend the active ingredient, lactose, and corn starch until uniform; then blend the magnesium stearate into the resulting powder. Encapsulate the mixture into suitably sized two-piece hard gelatin capsules.

Example 14

| Tablet | Amount (mg) | |
|---|---|---|
| Active Ingredient | 250.0 | 125.0 |
| Lactose 161.0 | 80.5 | |
| Corn Starch | 12.0 | 6.0 |
| Water (per thousand tablets) | 120. ml (evaporates) | 60. ml (evaporates) |
| Corn Starch | 75.0 | 37.5 |
| Magnesium Stearate | 2.0 | 1.0 |
| | 500.00 | 250.0 |

Blend the active ingredient with the lactose until uniform. Blend the smaller quantity of corn starch with the water and add the resulting corn starch paste, then mix until a uniform wet mass is formed. Add the remaining corn starch to the remaining wet mass and mix until uniform granules are obtained. Screen the granules through a suitable milling machine, using a 19.1 mm (3/4 inch) stainless steel screen. Dry the milled granules in a suitable drying oven until the desired moisture content is obtained. Mill the dried granules through a suitable milling machine using a 16 mesh stainless steel screen. Blend in the magnesium stearate and compress the resulting mixture into tablets of desired shape, thickness, hardness and disintergration.

Example 15

| Injectable Solution | mg/ml |
|---|---|
| Active ingredient | 5.00 |
| Methyl *p*-hydroxybenzoate | 0.80 |
| Propyl *p*-hydroxybenzoate | 0.10 |
| Disodium Edetate | 0.10 |
| Citric Acid Monohydrate | 0.08 |
| Dextrose 40.0 | |
| Water for injection qs. ad. | 1.0 ml |

Dissolve the *p*-hydroxybenzoates in a portion of water for injection at 60—70°C and cool the solution to 25—25°C. Charge and dissolve all other excipients and the active ingredient. Bring the solution to final volume, filter it through a sterilizing membrane and fill into sterile containers.

Similarly, substitute other compounds of the present invention to prepare other compositions of the present invention.

Example 16

| Topical Ophthamological Solution | mg/ml |
|---|---|
| Active Ingredient | 10.0 |
| Dibasic Sodium Phosphate | 10.4 |
| Monobasic Sodium Phosphate | 2.4 |
| Chlorobutanol | 5.0 |
| Hydroxypropyl methylcellulose | 5.0 |
| Sterile Water | q.s. ad 1.0 ml |
| 1.0H NaOH | q.s. ad pH 7.4 |

Mix the ingredients under sterile conditions and using standard techniques to obtain the ophthamological solution.

**Claims**

1. A compound of the general formula I

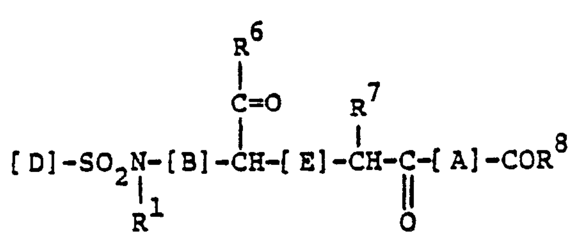

I

and the pharmaceutically acceptable salts thereof, wherein:

A is

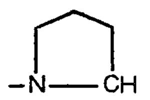 , 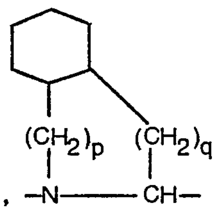 , 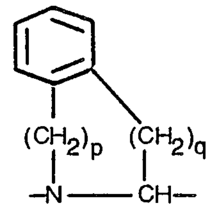 , 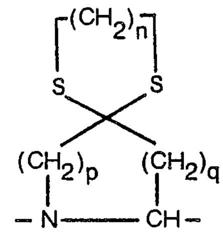 or 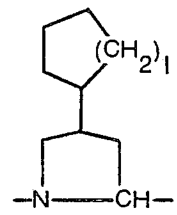

IIa IIb IIc IId IIe

l is 1 or 2;
n is 0 or 1;
p and q are 0, 1 or 2, provided that in structures IIb and IIc the sum of p and q is 1 or 2, and that in formula IId, p is not 0;
B is $-[J]-[L]-[M]-$;
D is

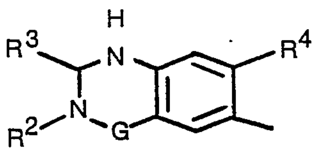

III

E is $-NH-$, $-O-$, $-S-$, or $-CH_2-$;

G is $-\overset{O}{\overset{\|}{C}}-$ or $-SO_2-$;

J is $-(CH_2)_s-$ or $-((:CH_2)_t-W)-$;
L is a chemical bond, cis- or trans $C_2-C_6$-alkenylene, $C_2-C_6$-alkynylene, —Z-aryl-, -aryl-Z-, —Z-cycloalkyl-, -cycloalkyl-Z-, a 5- or 6-membered heterocyclic radical comprising 3 to 5 carbon atoms and 1 or 2 hetero atoms selected from N, O and S, or a $R^5$-substituted heterocyclic radical, having one or more heteroatoms selected from N, O or S, wherein aryl is

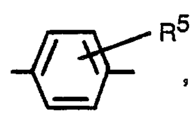 , 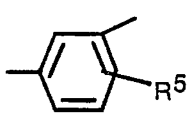 or 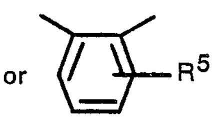

and cycloalkyl is

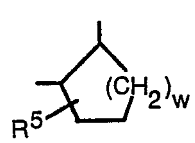 , 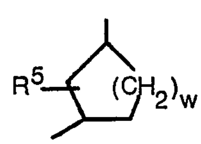 or 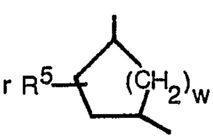

wherein
w is 1, 2 or 3;
M is $(CH_2)_u-$ or $-((CH_2)_t-X-(CH_2)_v)-$;
W is

$$-\overset{O}{\overset{\|}{C}}NH- \text{ or } -NH\overset{O}{\overset{\|}{C}}-;$$

X and Z are independently a chemical bond, $-NR^9-$, $-O-$, $-S-$,

$$-\overset{O}{\overset{\|}{C}}NH- \text{ or } -NH\overset{O}{\overset{\|}{C}}-;$$

s, u and v are independently 0—5;

t is 1—5;

$R^1$, $R^2$ and $R^9$ are independently hydrogen, $C_1$—$C_6$-alkyl or $C_1$—$C_6$-acyl;

$R^3$ is hydrogen, $C_1$—$C_6$-alkyl, halo-$C_1$—$C_6$-alkyl, phenyl-$C_1$—$C_6$-alkyl, (cycloalkyl)-$C_1$—$C_6$-alkyl, aminomethyl, $C_1$—$C_6$-alkylaminomethyl, phenyl-$C_1$—$C_6$-alkylaminomethyl, (cycloalkyl)-$C_1$—$C_6$-alkylaminomethyl, $C_1$—$C_6$-alkylthiomethyl, halo-$C_1$—$C_6$-alkylthiomethyl, 2-, 3- or 4-pyridyl-$C_1$—$C_6$-alkyl, 2-, 4- or 5-thiazolyl-$C_1$—$C_6$-alkyl, 2-, 4- or 5-1*H*-imidazolyl $C_1$—$C_6$-alkyl, 1-imidazolyl-$C_1$—$C_6$-alkyl, 1-morpholino-$C_1$—$C_6$-alkyl or hydroxyphenyl-$C_1$—$C_6$-alkyl;

$R^4$ is chlorine or $CF_3$;

$R^5$ is hydrogen, halogen, $C_1$—$C_6$-alkyl, $C_1$—$C_6$-acyl, $C_1$—$C_6$-alkoxy, halo-$C_1$—$C_6$-alkyl or phenyl-$C_1$—$C_6$-alkyl;

$R^7$ is hydrogen, alkyl or amino-$C_1$—$C_6$-alkyl;

$R^6$ and $R^8$ are independently hydroxy, alkoxy having from 1 to 8 carbon atoms, benzyl, allyl, $R^{10}$-$Q_r$—$(CH_2)_m$—O—, wherein Q is oxygen or sulfur, r is O or 1 and m is 2 to 4,

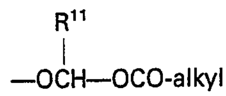

wherein the alkyl has from 3 to 8 carbon atoms,

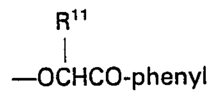

wherein the phenyl may be substituted with group T defined below, 1-glyceryl,

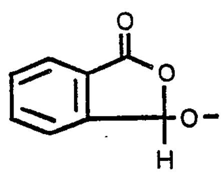

or

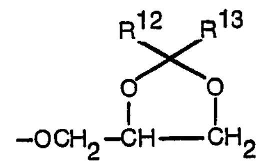

$R^{10}$ is phenyl, substituted phenyl wherein the substituents are chosen from group T, 1-naphthyl or 2-naphthyl;

T is halogen, hydroxy, trifluoromethyl, $C_1$—$C_6$-alkoxy, $C_1$—$C_6$-alkyl, 2-furanyl, 3-furanyl, 2-thienyl, 3-thienyl, phenyl and substituted phenyl wherein the substituents are chosen from halogen, hydroxy, trifluoromethyl, $C_1$—$C_6$-alkoxy or $C_1$—$C_6$-alkyl;

$R^{11}$ is hydogen or alkyl having from 1 to 8 carbon atoms;

$R^{12}$ is hydrogen, $C_1$—$C_6$-alkyl, unsubstituted or substituted phenyl and substituted or unsubstituted phenyl $C_1$—$C_6$-alkyl wherein phenyl may be substituted by group T;

$R^{13}$ is hydrogen or $C_1$—$C_6$-alkyl;

provided that if L is alkenylene alkynylene or, J is —$(CH_2)_s$— wherein s is 1—5; provided that if L is —Z-aryl- or —Z-cycloalkyl-, J is —$(CH_2)_s$— wherein s is 2—5; provided that if L is alkenylene alkynylene -aryl-Z- or -cycloalkyl-Z-, M is —$(CH_2)_u$- wherein u is 1—5; provided that if s and u are each zero, L is aryl or cycloalkyl (i.e. Z is a chemical bond); and provided that if s and v are each zero, L is aryl or cycloalkyl (i.e. Z is a chemical bond).

2. A compound as claimed in claim 1, wherein L is a chemical bond, cis- or trans-$C_2$—$C_6$-alkenylene, $C_2$—$C_6$-alkylene, —Z-aryl-, -aryl-Z-, —Z-cycloalkyl-, or -cycloalkyl-Z-, where aryl is

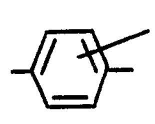

,

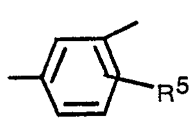

or

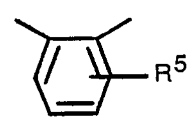

and cycloalkyl is

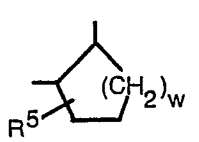

,

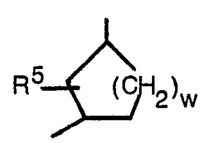

or

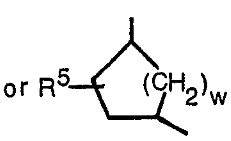

wherein

w is 1, 2 or 3, and

$R^3$ is hydrogen, $C_1$—$C_6$-alkyl, halo-$C_1$—$C_6$-alkyl, phenyl-$C_1$—$C_6$-alkyl, (cycloalkyl)-$C_1$—$C_6$-alkyl, amino-

methyl, $C_1$—$C_6$-alkylaminomethyl, phenyl-$C_1$—$C_6$-alkylaminomethyl, or (cycloalkyl)-$C_1$—$C_6$-alkylaminomethyl, $C_1$—$C_6$-alkylthiomethyl halo-$C_1$—$C_6$-alkylthiomethyl,
the remaining substituents being as defined for formula I.

3. A compound as claimed in claim 1 or claim 2, wherein A is of the formula IIa, IIb wherein p is zero and q is 1, or IId wherein p and q are each 1 and n is zero.

4. A compound as claimed in any one of the preceding claims, wherein D is of the formula III wherein G is —$SO_2$— and $R^4$ is chlorine.

5. A compound as claimed in claim 4, wherein in the moiety D, $R^2$ is hydrogen and $R^3$ is phenylethyl (cyclopentyl)methyl, chloromethyl, dichloromethyl, 2-pyridinylethyl, butyl, pentyl or trifluoroethylthiomethyl.

6. A compound as claimed in any one of the preceding claims, wherein E is —NH—.

7. A compound as claimed in any one of the preceding claims, wherein $R^7$ is hydrogen, methyl or aminobutyl and $R^6$ and $R^8$ are hydroxy.

8. A compound as claimed in any one of the preceding claims wherein B is of the formula:

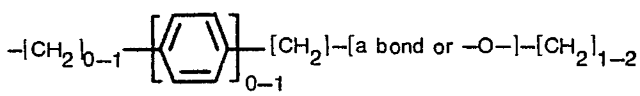

9. A compound as claimed in claim 1 selected from:
1-[[2-(S)-[[1(S)-carboxy-5-[[6-chloro-3-chloromethyl-3,4-dihydro-2*H*-1,2,4-benzothiadiazin-7-yl]-sulfonylamino]pentyl]amino]-1-oxopropyl]-[2S-(2α,3aα,7aα)]-octahydro-1*H*-indole-2-carboxylic acid, *S,S*-dioxide,
1-[2-(S)-[[1-(S)-carboethoxy-2-[4[[[6-chloro-3,4-dihydro-3-(2-phenylethyl)-2*H*-1,2,4-benzothiadiazin-7-yl]sulfonylamino]methyl]phenylmethoxy]ethyl]amino]-1-oxopropyl]-[2S-[2α,3aα,7aα)-octahydro-1*H*-indole-2-carboxylic acid, *S,S*-dioxide,
1-[2-(S)-[[1-(S)-carboxy-2-[4-[[[6-chloro-3,4-dihydro-3-(2-phenylethyl)-2*H*-1,2,4-benzothiadiazin-7-yl]-sulfonyl]amino]methyl]phenylmethoxy]ethyl]amino]-1-oxopropyl]-[2S-2α,3aα,7aα)]-octahydro-1*H*-indole-2-carboxylic acid, *S,S*-dioxide,
N-[2-(*S*)-[[3-[4-[[[6-chloro-3,4-dihydro-3-(2-phenylethyl)-2*H*-1,2,4-benzothiadiazin-7-yl]sulfonylamino]-methyl]phenyl]-1-(*S*)-(ethoxycarbonyl)butyl]amino]-1-oxopropyl]-(*S*)-proline-S,S-dioxide, and
1-(*N*)-[3-[4-[[[6-chloro-3,4-dihydro-3-(2-pyridylethyl)-2*H*-1,2,4-benzothiadiazin-7-yl]-sulfonyl]aminoethyl]phenyl]-1(*S*)-(ethoxycarbonyl)-propyl]-(*S*)-alanyl]-cis,syn-octahydroindole-2(*S*)-carboxylic acid S,S-dioxide.

10. A process for the preparation of a compound as claimed in claim 1, in which process the symbols A, B, D, E, J, L, $R^1$, $R^4$, $R^6$, $R^7$, $R^8$, t and v, unless otherwise stated, are as defined in claim 1, which process comprises:

A: condensing an acid of the general formula IV, or a reactive derivative thereof,

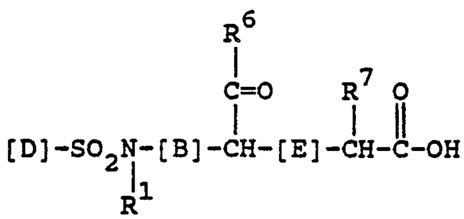

(IV)

with an amino acid of the general formula V, or a reactive derivative thereof,

$$H[A]—COR^8 \qquad (V), \text{ or}$$

B: condensing an amine of the general formula X,

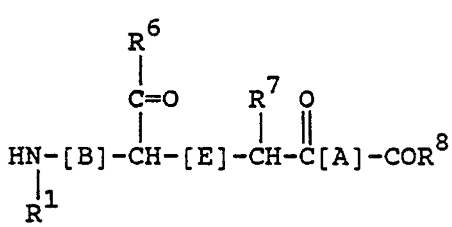

(X)

with a sulfonic acid of the general formula VIa, or a reactive derivative thereof,

$$[D].SO_2OH \qquad (VIa), \text{ or}$$

C: condensing an aminosulfonamide of the general formula XIV,

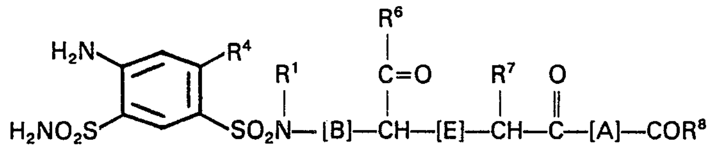

(XIV)

with an aldehyde of the general formula XIII: $R^3$—CHO, or a reactive derivative thereof, or

D: condensing a halide of the general formula XV

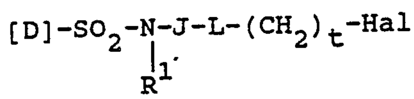

(XV),

where Hal represents halogen, with a thiol of the general formula XVI

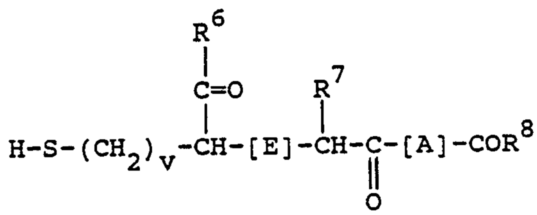

(XVI),

said process being followed, if desired, by a step of esterification or de-esterification, the so-obtained compound of the general formula I being isolated in free form or in the form of a pharmaceutically acceptable salt.

11. A pharmaceutical composition which comprises a compound as claimed in claim 1 in association with a pharmaceutically acceptable carrier.

12. A pharmaceutical composition as claimed in claim 11 in the form of topical opthalmologically acceptable composition for reducing and controlling elevated intraocular pressure associated with glaucoma.

13. A pharmaceutical composition as claimed in claim 11 or 12 in the form of dosage units.

**Patentansprüche**

1. Verbindung der allgemeinen Formel I

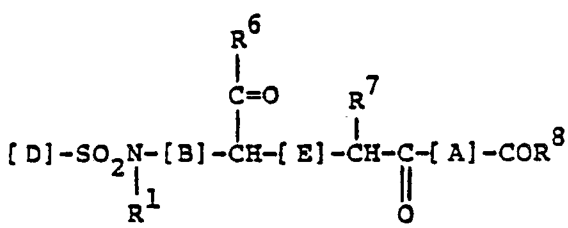

I

oder deren pharmazeutische Salze, worin

A

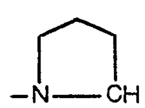

IIa

,

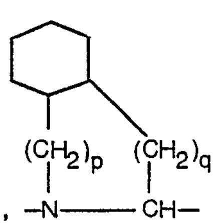

IIb

,

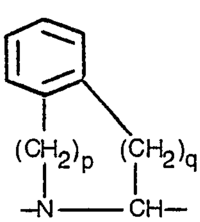

IIc

,

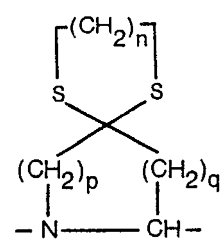

IId

oder

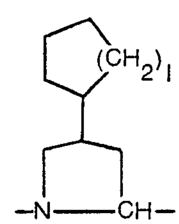

IIe

ist, l 1 oder 2 ist,

n 0 oder 1 ist,

p und q 0, 1 oder 2 sind, mit der Maßgabe, daß in den Strukturen IIb und IIc die Summe von p und q 1 oder 2 ist, und daß in Formel IId p nicht 0 ist,

B —[J]—[L]—[M]— ist,

D

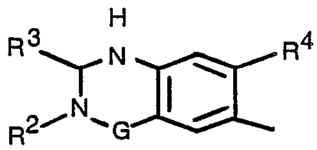 ist,

III

E —NH—, —O—, —S— oder —$CH_2$— ist,

G $-\overset{O}{\overset{\|}{C}}-$ oder —$SO_2$— ist;

J —$(CH_2)_s$— oder ($(CH_2)_t$—W)— ist,

L eine chemische Bindung, cis- oder trans-$C_2$—$C_6$-Alkenylen, $C_2$—$C_6$-Alkinylen, —Z-Aryl-, -Aryl-Z-, —Z-Cycloalkyl-, -Cycloalkyl-Z-, ein 5- oder 6-gliedriger, 3 bis 5 Kohlenstoff-Atome und 1 oder 2 aus N, O und S ausgewählte Hetero-Atome umfassender heterocyclischer Rest oder ein $R^5$-substituierter heterocyclischer Rest mit einem oder mehreren, aus N, O oder S ausgewählten Hetero-Atomen ist,

worin

Aryl

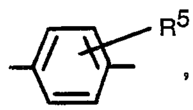 , 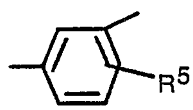 oder 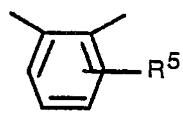

ist und

Cycloalkyl

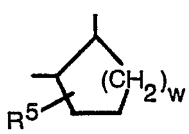 , 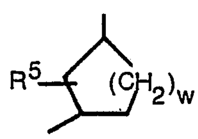 oder 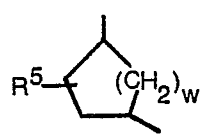 ist,

worin

w 1, 2 oder 3 ist,

M —$(CH_2)_u$— oder —($(CH_2)_t$—X—$(CH_2)_v$)— ist,

W

$-\overset{O}{\overset{\|}{C}}NH-$ oder $-NH\overset{O}{\overset{\|}{C}}-$ ist,

X und Z unabhängig voneinander eine chemische Bindung, —$NR^9$—, —O—, —S—,

$-\overset{O}{\overset{\|}{C}}NH-$ oder $-NH\overset{O}{\overset{\|}{C}}-$ sind,

s, u und v unabhängig voneinander 0 bis 5 sind,

t 1 bis 5 ist,

$R^1$, $R^2$ und $R^9$ unabhängig voneinander Wasserstoff, Niederalkyl oder Niederacyl sind,

$R^3$ Wasserstoff, $C_1$—$C_6$-Alkyl, Halogeno-$C_1$—$C_6$-alkyl, Phenyl-$C_1$—$C_6$-alkyl, (Cycloalkyl)-$C_1$—$C_6$-alkyl, Aminomethyl, $C_1$—$C_6$-Alkylaminomethyl, Phenyl-$C_1$—$C_6$-alkylaminomethyl, (Cycloalkyl)-$C_1$—$C_6$-alkylaminomethyl, $C_1$—$C_6$-Alkylthiomethyl, Halogeno-$C_1$—$C_6$-alkylthiomethyl, 2-, 3- oder 4-Pyridyl-$C_1$—$C_6$-alkyl, 2-, 4- oder 5-Thiazolyl-$C_1$—$C_6$-alkyl, 2-, 4- oder 5-1*H*-Imidazolyl-$C_1$—$C_6$-alkyl, 1-Imidazolyl-$C_1$—$C_6$-alkyl, 1-Morpholino-$C_1$—$C_6$-alkyl oder Hydroxyphenyl-$C_1$—$C_6$-alkyl ist,

$R^4$ Chlor oder $CF_3$ ist,

$R_5$ Wasserstoff, Halogen, $C_1$—$C_6$-Alkyl, $C_1$—$C_6$-Acyl, $C_1$—$C_6$-Alkoxy, Halogeno-$C_1$—$C_6$-alkyl oder Phenyl-$C_1$—$C_6$-alkyl ist,

$R^7$ Wasserstoff, $C_1$—$C_6$-Alkyl oder Amino-$C_1$—$C_6$-alkyl ist,

$R^6$ und $R^8$ unabhängig voneinander Hydroxy, Alkoxy mit 1 bis 8 Kohlenstoff-Atomen, Benzyl, Allyl, $R^{10}$—$Q_r$—$(CH_2)_m$—O—, worin Q Sauerstoff oder Schwefel ist, r 0 oder 1 ist und m 2 bis 4 ist,

$-O\overset{R^{11}}{\overset{|}{C}}H-OCO\text{-Alkyl-},$

worin das Alkyl 3 bis 8 Kohlenstoff-Atome hat,

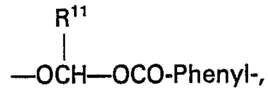

worin das Phenyl mit einer nachstehend definierten Gruppe T substituiert sein kann, 1-Glyceryl,

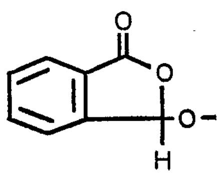

or

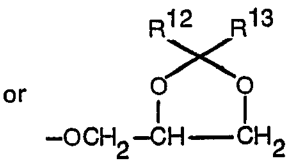

$R^{10}$ Phenyl, substituiertes Phenyl, worin die Subtituenten aus der Gruppe T gewählt sind, 1-Naphthyl oder 2-Naphthyl ist,

T Halogen, Hydroxy, Trifluoromethyl, Niederalkoxy, Niedralkyl, 2-Furanyl, 3-Furanyl, 2-Thienyl, 3-Thienyl, Phenyl und substituiertes Phenyl ist, worin die Substituenten aus Halogen, Hydroxy, Trifluoromethyl, $C_1$—$C_6$-Alkoxy und $C_1$—$C_6$-Alkyl ausgewählt sind,

$R^{11}$ Wasserstoff oder Alkyl mit 1 bis 8 Kohlenstoff-Atomen ist,

$R^{12}$ Wasserstoff, $C_1$—$C_6$-Alkyl, unsubstituierts oder substituiertes Phenyl und substituiertes oder unsubstituiertes Phenyl-$C_1$—$C_6$-alkyl ist, worin Phenyl durch die Gruppe T substitiert sein kann,

$R^{13}$ Wasserstoff oder $C_1$—$C_6$-Alkyl ist, mit der Maßgabe, daß, wenn L Alkenylen oder Alkinylen ist, J —$(CH_2)_s$— ist, worin s 1 bis 5 ist, mit der Maßgabe, daß, wenn L —Z-Aryl- oder —Z-Cycloalkyl- ist, J —$(CH_2)_s$— ist, worin s 2 bis 5 ist, mit der Maßgabe, daß, wenn L Alkenylen, Alkinylen, Aryl-Z- oder Cycloalkyl-Z- ist, M —$(CH_2)_u$— ist, worin u 1 bis 5 ist, mit der Maßgabe, daß, wenn s und u jeweils Null sind, L Aryl oder Cycloalkyl ist (d.h. Z eine chemische Bindung ist) und mit der Maßgabe, daß, wenn s und v jeweils Null sind, L Aryl oder Cycloalkyl ist (d.h. Z eine chemische Bindung ist).

2. Verbindung nach Anspruch 1, worin

L eine chemische Bindung, cis- oder trans-$C_2$—$C_6$-Alkenylen, $C_2$—$C_6$-Alkinylen, —Z-Aryl, -Aryl-Z-, —Z-Cycloalkyl- oder -Cycloalkyl-Z- ist,

worin

Aryl

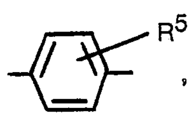

,

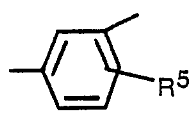

oder

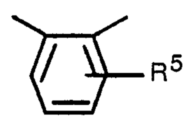

ist und

Cycloalkyl

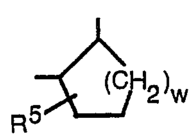

,

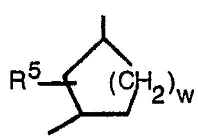

oder

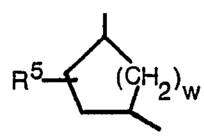

ist,

worin

w 1, 2 oder 3 ist und

$R^3$ Wasserstoff, $C_1$—$C_6$-Alkyl, Halogeno-$C_1$—$C_6$-alkyl, Phenyl-$C_1$—$C_6$-alkyl, (Cycloalkyl)-$C_1$—$C_6$-alkyl, Aminomethyl, $C_1$—$C_6$-Alkylaminomethyl, Phenyl-$C_1$—$C_6$-alkylaminomethyl oder (Cycloalkyl)-$C_1$—$C_6$-alkylaminomethyl, $C_1$—$C_6$-Alkylthiomethyl, Halogeno-$C_1$—$C_6$-alkylthiomethyl ist,

wobei die verbleibenden Substituenten die in Anspruch 1 angegebenen Bedeutungen haben.

3. Verbindung nach Anspruch 1 oder Anspruch 2, worin A die Formel IIa, IIb, worin p Null ist und q 1 ist oder IId, worin p und q jeweils 1 sind und n Null ist, hat.

4. Verbindung nach irgendeinem der vorhergehenden Ansprüche, worin D die Formel III hat, in der G —$SO_2$— ist und $R^4$ Chlor ist.

5. Verbindung nach Anspruch 4, worin in der Struktureinheit D $R^2$ Wasserstoff ist und $R^3$ Phenylethyl-(cyclopentyl)methyl, Chloromethyl, Dichloromethyl, 2-Pyridinylethyl, Butyl, Pentyl oder Trifluoroethylthiomethyl ist.

6. Verbindung nach irgendeinem der vorhergehenden Ansprüche, worin E —NH— ist.

7. Verbindung nach irgendeinem der vorhergehenden Ansprüche, worin $R^7$ Wasserstoff, Methyl oder Aminobutyl ist und $R^6$ und $R^8$ Hydroxy sind.

8. Verbindung nach irgendeinem der vorhergehenden Ansprüche, worin B die Formel

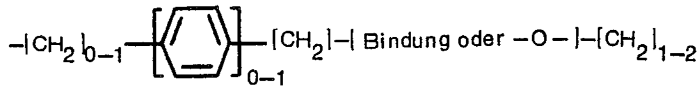 hat.

9. Verbindung nach Anspruch 1, ausgewählt aus

1-[[2-(S)-[[1-(S)-Carboxy-5-[[6-chloro-3-chloromethyl-3,4-dihydro-2*H*-1,2,4-benzothiadiazin-7-yl]-sulfonylamino]pentyl]amino]-1-oxopropyl]-[2S-(2α,3aα,7aα)]-octahydro-1*H*-indol-2-carbonsäure-S,S-dioxid,

1-[2-(S)-[[1-(S)-Carboethoxy-2-[4[[[6-chloro-3,4-dihydro-3-(2-phenylethyl)-2*H*-1,2,4-benzothiadiazin-7-yl]-sulfonylamino]methyl]phenylmethoxy]ethyl]amino]-1-oxopropyl]-[2S-[2α,3aα,7aα)-octahydro-1*H*-indol-2-carbonsäure-S,S-dioxid,

1-[2-(S)-[[1-(S)-Carboxy-2-[4-[[[6-chloro-3,4-dihydro-3-(2-phenylethyl)-2*H*-1,2,4-benzothiadiazin-7-yl]-sulfonyl]amino]methyl]phenylmethoxy]ethyl]amino]-1-oxopropyl]-[2S-(2α,3aα,7aα)]-octahydro-1*H*-indol-2-carbonsäure-S,S-dioxid,

N-[2-(S)-[[3-[4-[[[6-Chloro-3,4-dihydro-3-(2-phenylethyl)-2*H*-1,2,4-benzothiadiazin-7-yl]sulfonylamino]-methyl]phenyl]-1-(S)-(ethoxycarbonyl)butyl]amino]-1-oxopropyl]-(S)-prolin-S,S-dioxid und

1-(N)-[3-[4-[[[6-Chloro-3,4-dihydro-3-(2-pyridylethyl)-2*H*-1,2,4-benzothiadiazin-7-yl]-sulfonyl]aminoethyl]phenyl]-1(S)-(ethoxycarbonyl)-propyl]-(S)-alanyl]-cis,syn-octahydroindole-2(S)-carbonsäure-S,S-dioxid.

10. Verfahren zur Herstellung einer Verbindung nach Anspruch 1, worin die Symbole A, B, D, E, J, L, $R^1$, $R^4$, $R^6$, $R^7$, $R^8$, t und v, soweit nichts anderes angegeben ist, die in Anspruch 1 festgelegten Bedeutungen haben umfassend

A: die Kondensation einer Säure der allgemeinen Formel IV oder eines reaktionsfähigen Derivatives derselben

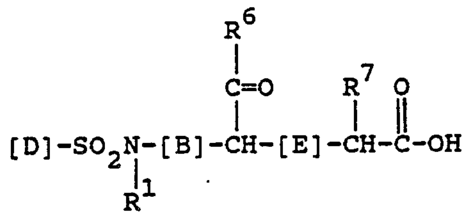 (IV)

mit einer Aminosäure der allgemeinen Formel V oder einem reaktionsfähigen Derivat derselben

$$H[A]—COR^8 \qquad (V)$$

oder

B: die Kondensation eines Amins der allgemeinen Formel X

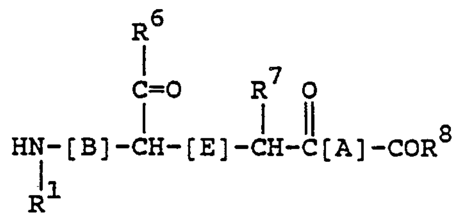 (X)

mit einer Sulfonsäure der allgemeinen Formel VIa oder einem reaktionsfähigen Derivat derselben

$$[D].SO_2OH \qquad (VIa)$$

oder

C: die Kondensation eines Aminosulfonamids der allgemeinen Formel XIV

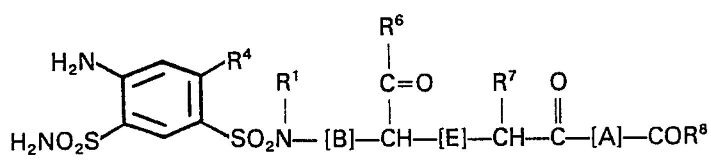 (XIV)

mit einem Aldehyd der allgemeinen Formel XIII: $R^3$—CHO, oder einem reaktionsfähigen Derivat desselben oder

D: die Kondensation eines Halogenids der allgemeinen Formel XV

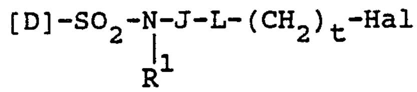 (XV),

worin Hal Halogen bezeichnet, mit einem Thiol der allgemeinen Formel XVI

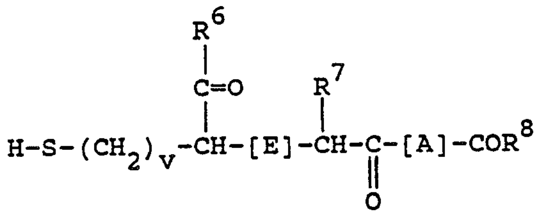 (XVI),

wobei auf diesen Schritt gewünschtenfalls ein Schritt der Veresterung oder der Ester-Spaltung folt und die so erhaltene Verbindung der Formel I in freier Form oder in Form eines pharmazeutisch annehmbaren Salzes isoliert wird.

11. Pharmazeutische Zusammensetzung, umfassend eine Verbindung nach Anspruch 1 in Verbindung mit einem pharmazeutisch geeigneten Träger.

12. Pharmazeutische Zusammensetzung nach Anspruch 11 in Form einer topischen, ophthalmologisch annehmbaren Zusammensetzung zur Reduzierung und Regulierung eines erhöhten Augeninnendrucks im Zusammenhang mit einem Glaukom.

13. Pharmazeutische Zusammensetzung nach Anspruch 11 oder Anspruch 12 in Form von Dosierungseinheiten.

**Revendications**

1. Composé de la formule générale I

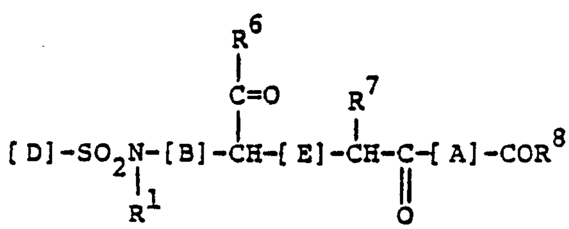 I

et ses sels acceptables en pharmacie, où:

A est

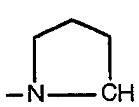 , 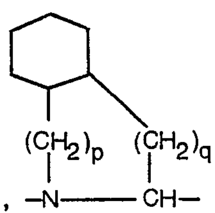 , 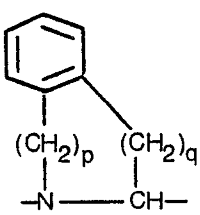 , 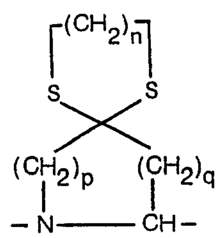 ou 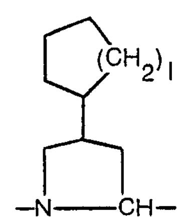

IIa IIb IIc IId IIe

l est 1 ou 2;

n est 0 ou 1;

p et q sont 0, 1 ou 2 à condition que dans les structures IIb et IIc, la somme de p et q soit 1 ou 2 et que dans la formule IId, p ne soit pas 0;

B est —[J]—[L]—[M]—;

D est

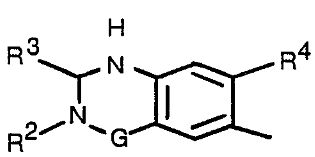

III

E est —NH—, —O—, —S—, ou —$CH_2$—;

G est $-\overset{O}{\overset{\|}{C}}-$; ou —$SO_2$—;

J est —$(CH_2)_s$— ou —($(CH_2)_t$—W)—;

L est une liaison chimique, cis-ou trans-alkénylène $C_2$—$C_6$, alkynylène $C_2$—$C_6$, —Z-aryl-, -aryl-Z-, —Z-cycloalkyl-, -cycloalkyl-Z-, un radical hétérocyclique à 5 ou 6 membres comprenant 3 à 5 atomes de carbone et 1 ou 2 hétéroatomes choisis parmi N, O et S, ou bien un radical hétérocyclique $R^5$-substitué, ayant un ou plusieurs hétéroatomes choisis parmi N, O ou S, où aryle est

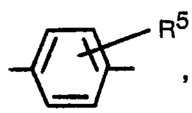 , 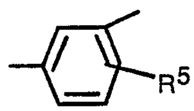 ou 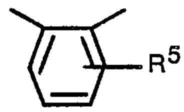

et cycloalcoyle est

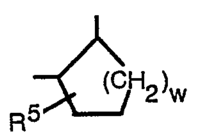 , 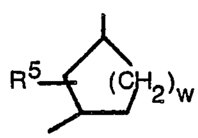 ou 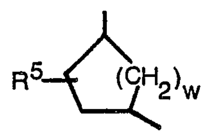 où

w est 1, 2 ou 3;

M est —$CH_2)_u$— ou —($(CH_2)_t$—X—$(CH_2)_v$)—;

W est

$-\overset{O}{\overset{\|}{C}}NH-$ ou $-NH\overset{O}{\overset{\|}{C}}-$;

X et Z sont indépendamment une liaison chimique, —$NR^9$—, —O—, —S—,

$-\overset{O}{\overset{\|}{C}}NH-$ ou $-NH\overset{O}{\overset{\|}{C}}-$;

s, u et v sont indépendamment 0—5;

t est 1—5;

$R^1$, $R^2$ et $R^9$ sont indépendamment hydrogène, alcoyle inférieur ou acyl inférieur;

$R^3$ est hydrogène, alcoyle $C_1$—$C_6$, haloalcoyle $C_1$—$C_6$, phénylalcoyle $C_1$—$C_6$, (cycloalkyl)alcoyle $C_1$—$C_6$, aminométhyle, alkyl $C_1$—$C_6$-aminométhyle, phénylalkyl $C_1$—$C_6$-aminométhyle, (cycloalkyl)alkyl $C_1$—$C_6$-aminométhyle, alkyl $C_1$—$C_6$-thiomèthyle, haloalkyl $C_1$—$C_6$-thiomèthyle, 2-, 3- ou 4-pyridylalcoyle $C_1$—$C_6$, 2-, 4- ou 5-thiazolylalcoyle $C_1$—$C_6$, 2-, 4- ou 5-1*H*-imidazolylalcoyle $C_1$—$C_6$, 1-imidazolylalcoyle $C_1$—$C_6$, 1-morpholinoalcoyle $C_1$—$C_6$ ou hydroxyphénylalcoyle $C_1$—$C_6$;

$R^4$ est chlore ou $CF_3$;

$R^5$ est hydrogène, halogène, alcoyle $C_1$—$C_6$, acyle $C_1$—$C_6$, alcoxy $C_1$—$C_6$, haloalcoyle $C_1$—$C_6$ ou phényl-alcoyle $C_1$—$C_6$;

$R^7$ est hydrogène, alcoyle ou aminoalcoyle $C_1$—$C_6$;

$R^6$ et $R^8$ sont idnépendamment hydroxy, alcoxy ayant de 1 à 8 atomes de carbone, benzyle, allyle, $R^{10}$—$Q_r$—$(CH_2)_m$—O— où Q est oxygène ou soufre, r est 0 ou 1 et m est 2 à 4,

$-O\overset{R^{11}}{\overset{|}{C}}H-OCO$-alcoyle,

où l'alcoyle à de 3 à 8 atomes de carbone,

$-O\overset{R^{11}}{\overset{|}{C}}HCO$-phényle

où le phényle peut être substitué par un groupe T défini ci-dessous, 1-glycéryle,

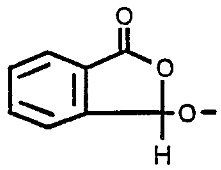 ou 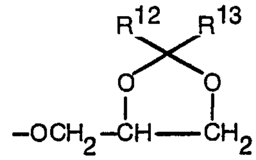

$R^{10}$ est phényle, phényle substitué où les substituants sont choisis parmi le groupe T, 1-naphtyle ou 2-naphtyle;

T est halogène, hydroxy, trifluorométhyle, alcoxy inférieur, alcoyle inférieur, 2-furanyle, 3-furanyle, 2-thiényle, 3-thiényle, phényle et phényle substitué où les substituants sont choisis parmi halogène, hydroxy, trifluorométhyle, alcoxy $C_1$—$C_6$ ou alcoyle $C_1$—$C_6$;

$R^{11}$ est hydrogène ou alcoyle ayant de 1 à 8 atomes de carbone;

$R^{12}$ est hydrogène, alcoyle $C_1$—$C_6$, phényle non substitué ou substitué et phénylalcoyle $C_1$—$C_6$ non substitué ou substitué, où phényle peut être substitué par le groupe T;

$R^{13}$ est hydrogène ou alcoyle $C_1$—$C_6$;

à condition qui si L est alkénylène ou alkynylène, J soit —$(CH_2)_s$— où s est 1—5; à condition qu si L est —Z-aryl- ou Z-cycloalkyl-, J soit —$(CH_2)_s$ où s est 2—5; à condition que si L est alkénylène, alkynylène, -aryl-Z- ou -cycloalkyl-Z-, M soit —$(CH_2)_u$— où u est 1—5; à condition que si s et u sont chacun zéro, L soit aryle ou cycloalcoyle (i.e. Z est une liaison chimique); et à condition que si s et v sont chacun zéro, L soit aryle ou cycloalcoyle (i.e. Z est une liaison chimique).

2. Composé selon la revendication 1, où L est une liaison chimique, cis- ou trans-alkénylène $C_2$—$C_6$, alkynylène $C_2$—$C_6$, —Z-aryl-, -aryl-Z—, —Z-cycloalkyl- ou -cycloalkyl-Z—, où aryle est

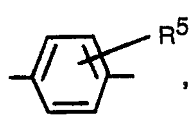, 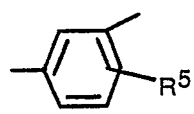 ou 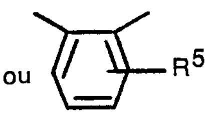

et cycloalcoyle est

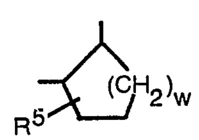, 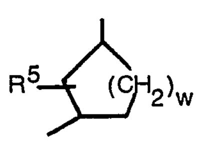 ou 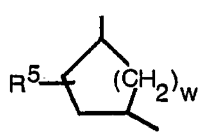 où

w est 1, 2 ou 3 et

$R^3$ est hydrogène, alcoyle $C_1$—$C_6$, haloalcoyle $C_1$—$C_6$, phénylalcoyle $C_1$—$C_6$, (cycloalkyl)alcoyle $C_1$—$C_6$, aminométhyle, alkyl $C_1$—$C_6$-aminométhyle, phénylalkyl $C_1$—$C_6$-aminométhyle ou (cycloalkyl)alkyl $C_1$—$C_6$-aminométhyle, alkyl $C_1$—$C_6$-thiométhyle, halalkyl $C_1$—$C_6$-thiométhyle,

les substituants restants étant tels que définis pour la formule I.

3. Composé selon la revendication 1 ou la revendication 2, où A est de la formule IIa, IIb où p est zèro et q est 1, ou bien IId où p et q sont chacun 1 et n est zéro.

4. Composé selon l'une quelconque des revendications précédentes, où D est de la formule III où G est —$SO_2$— et $R^4$ est chlore.

5. Composé selon la revendication 4, où dans la fragment D, $R^2$ est hydrogène et $R^3$ est phényléthyle (cyclopentyl)méthyle, chlorométhyle, dichlorométhyle, 2-pyridinyléthyle, butyle, pentyle ou trifluoroéthyl-thiométhyle.

6. Composé selon l'une quelconque des revendications précédentes, où E est —NH—.

7. Composé selon l'une quelconque des revendications précédentes, où $R^7$ est hydrogène, méthyle ou aminobutyle et $R^6$ et $R^8$ sont hydroxy.

8. Composé selon l'une quelconque des revendications précédentes, où B est de la formule

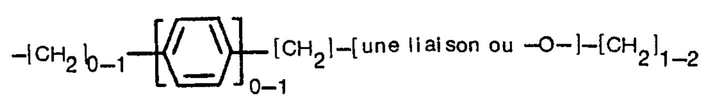


9. Composé selon la revendication 1 choisi parmi:

acide 1-[[2-(S)-[[1(S)-carboxy-5-[[6-chloro-3-chlorométhyl-3,4-dihydro-2*H*-1,2,4-benzothiadiazin-7-yl]-sulfonylamino]pentyl]amino]-1-oxopropyl]-[2S-(2α,3aα,7aα)]-octahydro-1*H*-indole-2-carboxylique, *S,S*-dioxyde,

acide 1-[2-(S)-[[1-(S)-carboéthoxy-2-[4[[[6-chloro-3,4-dihydro-3-(2-phényléthyl)-2*H*-1,2,4-benzothia-diazin-7-yl]sulfonylamino]méthyl]phénylméthoxy]éthyl]amino]-1-oxopropyl]-[2S-[2α,3aα,7aα)-octahydro-1*H*-indole-2-carboxylique, *S,S*-dioxyde,

acide 1-[2-(S)-[[1-(S)-carboxy-2-[4-[[[6-chloro-3,4-dihydro-3-(2-phényléthyl)-2*H*-1,2,4-benzothiadiazin-7-yl]sulfonyl]amino]méthyl]phénylméthoxy]éthyl]amino]-1-oxopropyl]-[2S-2α,3aα,7aα)]-octahydro-1*H*-indole-2-carboxylique, *S,S*-dioxyde,

N-[2-(*S*)-[[3-[4-[[[6-chloro-3,4-dihydro-3-(2-phényléthyl)-2*H*-1,2,4-benzothiadiazin-7-yl]sulfonylamino]-méthyl]phényl]-1-(*S*)-(éthoxycarbonyl)butyl]amino]-1-oxopropyl]-(*S*)-proline-S,S-dioxyde, et

acide 1-(*N*)-[3-[4-[[[6-chloro-3,4-dihydro-3-(2-pyridyléthyl)-2*H*-1,2,4-benzothiadiazin-7-yl]-sulfonyl]-aminoéthyl]phényl]-1(*S*)-(éthoxycarbonyl)-propyl]-(*S*)-alanyl]-cis,syn-octahydroindole-2(*S*)-carboxylique, S,S-dioxyde.

10. Procédé pour le préparation d'un composé selon la revendication 1, procédé dans lequel les symboles A, B, D, E, J, L, $R^1$, $R^4$, $R^6$, $R^7$, $R^8$, t et v, à moins que cela ne soit indiqué autrement, sont tels que définis à la revendication 1, lequel procédé comprend:

A: la condensation d'un acide de la formule générale IV, ou son dérivé réactif,

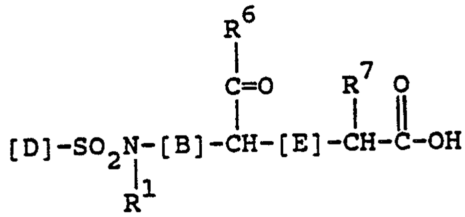 (IV)

avec un aminoacide de la formule générale V, ou son dérivé réactif,

$$H[A]—COR^8$$ (V), ou

B: la condensation d'une amine de la formule générale X,

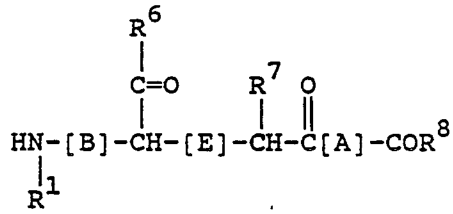 (X)

avec un acide sulfonique de la formule générale VIa, ou son dérivé réactif,

$$[D].SO_2OH$$ (VIa), ou

C: la condensation d'un aminosulfonamide de la formule générale XIV,

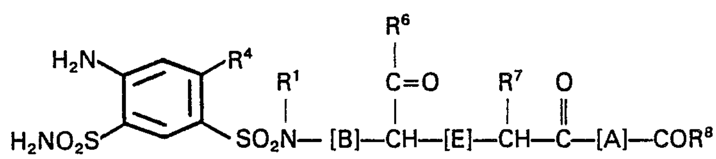 (XIV)

avec un aldéhyde de la formule générale XIII: $R^3$—CHO ou son dérivé réactif ou

D: la condensation d'un halogénure de la formule générale XV

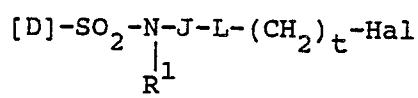 (XV),

où Hal représente halogène, avec un thiol de la formule générale XVI

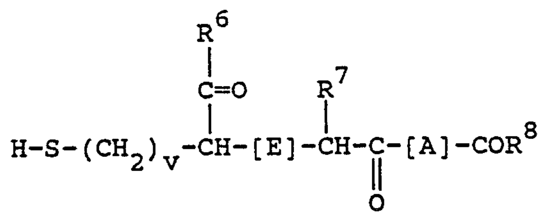 (XVI),

ledit procédé étant suivi, si on le souhaite, d'une étape d'estérification ou de dé déstérification, le composé ainsi obtenu de la formule générale I étant isolé sos forme libre ou sous la forme d'un sel acceptable en pharmacie.

11. Composition pharmaceutique qui comprend un composé selon la revendication 1 en association avec un véhicule acceptable en pharmacie.

12. Composition pharmaceutique selon la revendication 11 sous la forme d'une composition topique ophtalmogiquement acceptable pour réduire et contrôler une pression intra-oculaire élevée associée au glaucome.

13. Composition pharmaceutique selon la revendication 11 ou la revendication 12 sous la forme de doses unitaires.